# EUROPEAN PATENT SPECIFICATION

(11) **EP 3 845 642 B1**
(45) Date of publication and mention of the grant of the patent: **09.08.2023**
(21) Application number: 20209908.1
(22) Date of filing: 25.04.2017
(51) Int. Cl.: C12N 9/54, C11D 3/386

(54) **PROTEASE VARIANTS AND USES THEREOF**
PROTEASEVARIANTEN UND VERWENDUNGEN DAVON
VARIANTES DE PROTÉASE ET LEURS UTILISATIONS

(30) Priority: 05.05.2016 US 201662332417 P
(43) Date of publication of application: 07.07.2021
(62) Divisional of application: 17722562.0
(73) Proprietor: Danisco US Inc., Palo Alto, CA 94304 (US)
(72) Inventor: AMIN, Neelam, S., Palo Alto, CA 94304 (US); AUGUSTYN, Katherine, Palo Alto, CA 94304 (US); CHANDRASEKARAN, Priyanka, Palo Alto, CA 94304 (US); LEE, Edwin, W., Palo Alto, CA 94304 (US)
(74) Representative: Mewburn Ellis LLP

(56) References cited:
- WO-A1-2015/038792
- JP-A- 2003 325 186
- DATABASE UniProt [Online] 5 July 2004 (2004-07-05), "SubName: Full=Protease {ECO:0000313|EMBL:BAD02409.1};", XP002771514, retrieved from EBI accession no. UNIPROT:Q76L29 Database accession no. Q76L29 -& KATSUHISA SAEKI ET AL: "Nucleotide and Deduced Amino Acid Sequences of a New Subtilisin from an Alkaliphilic Bacillus Isolate", CURRENT MICROBIOLOGY, vol. 47, no. 4, 1 October 2003 (2003-10-01), pages 337-340, XP055385183, Boston ISSN: 0343-8651, DOI: 10.1007/s00284-002-4018-9

## Description

Disclosed herein is one or more subtilisin variant useful for cleaning applications and in methods of cleaning, as well as in a variety of industrial applications. One embodiment is directed to one or more subtilisin variant, including one or more *Bacillus sp.* subtilisin polypeptide variant, and one or more cleaning composition comprising one or more such variant.

Serine proteases are enzymes (EC No. 3.4.21) possessing an active site serine that initiates hydrolysis of peptide bonds of proteins. Serine proteases comprise a diverse class of enzymes having a wide range of specificities and biological functions that are further divided based on their structure into chymotrypsin-like (trypsin-like) and subtilisin-like. The prototypical subtilisin (EC No. 3.4.21.62) was initially obtained from *Bacillus subtilis.* Subtilisins and their homologues are members of the S8 peptidase family of the MEROPS classification scheme. Members of family S8 have a catalytic triad in the order Asp, His and Ser in their amino acid sequence. Although a number of useful variant proteases have been developed for cleaning applications, there remains a need for improved protease variants.

JP 2003 3325 186 A describes an alkaline protease useful as a detergent-blending enzvmc and a gene encoding the same_{.}

KATSUHISA SAEKI ET AL: "Nucleotide and Deduced Amino Acid Sequences of a New Subtilisin from an Alkaliphilic Bacillus Isolate", CURRENT MICROBIOLOGY, vol. 47, no. 4, 1 October 2003 (2003-10-01), pages 337-340, XP055385183 discloses a subtilisin KSM-LD1 BAD02409_{.}1 from an alkaliphic Bacillus isolate.

WO 2015/038792 A1 discloses serine protease enzymes produces from LG12-clade enzymes and compositions comprising such enzymes.

The invention provides subtilisin variants as set out in the claims. Some embodiments of the invention are directed to a composition comprising one or more subtilisin variants as set out in the claims. Further embodiments are directed to a method of cleaning comprising contacting a surface or an item in need of cleaning with one or more subtilisin variant or one or more compositions as set out in the claims.

Figures 1A-1C provide a MUSCLE multiple sequence alignment of BspE04637-T1 and variants thereof with the truncated form of BspE04637; previously disclosed BspE04637-clade subtilisins: SWT183_1430046 and *Bacillus sp.* KSM-LD1_ BAD02409.1; and the sequences of the mature forms of the following subtilisins: *B. amyloliquefaciens_*CAA24990, *B. lentus_*P29000*, Bacillus sp.* SprC_AAC43580, and *B. licheniformis*_CAJ7073*.*

Figure 2 provides a phylogenetic tree of subtilisins including BspE04637 and variants thereof.

Unless otherwise indicated herein, the subtilisin variants described herein can be made and used via conventional techniques commonly used in molecular biology, microbiology, protein purification, protein engineering, protein and DNA sequencing, recombinant DNA fields, and industrial enzyme use and development. Terms and abbreviations not defined should be accorded their ordinary meaning as used in the art. Unless defined otherwise herein, all technical and scientific terms used herein have the same meaning as commonly understood by one of ordinary skill in the art. Any definitions provided herein are to be interpreted in the context of the specification as a whole. As used herein, the singular "a," "an" and "the" includes the plural unless the context clearly indicates otherwise. Unless otherwise indicated, nucleic acid sequences are written left to right in 5' to 3' orientation; and amino acid sequences are written left to right in amino to carboxy orientation. Each numerical range used herein includes every narrower numerical range that falls within such broader numerical range, as if such narrower numerical ranges were all expressly written herein.

As used herein in connection with a numerical value, the term "about" refers to a range of +/- 0.5 of the numerical value, unless the term is otherwise specifically defined in context. For instance, the phrase a "pH value of about 6" refers to pH values of from 5.5 to 6.5, unless the pH value is specifically defined otherwise.

The amino acid substitutions of the one or more subtilisin variant described herein is described using one or more of the following nomenclatures: position or starting amino acid(s):position:substituted amino acid(s). Reference to only a position encompasses any starting amino acid that may be present in a reference subtilisin at that position and any amino acid with which such starting amino acid may be substituted (i.e., the substituted amino acid necessarily excludes the starting amino acid of such reference subtilisin). Reference to a starting or substituted amino acid may be further expressed as several starting or substituted amino acids separated by a forward slash ("/"). For example, D275S/K indicates position 275 is substituted with serine (S) or lysine (K) and P/S197K indicates that starting amino acid proline (P) or serine (S) at position 197 is substituted with lysine (K).

The terms "protease" and "proteinase" refer to an enzyme that has the ability to break down proteins and peptides. A protease has the ability to conduct "proteolysis," by hydrolysis of peptide bonds that link amino acids together in a peptide or polypeptide chain forming the protein. This activity of a protease as a protein-digesting enzyme is referred to as "proteolytic activity." Many well-known procedures exist for measuring proteolytic activity. For example, proteolytic activity may be ascertained by comparative assays that analyze the respective protease's ability to hydrolyze a suitable substrate. Exemplary substrates useful in the analysis of protease or proteolytic activity, include, but are not limited to, di-methyl casein (Sigma C-9801), bovine collagen (Sigma C-9879), bovine elastin (Sigma E-1625), and bovine keratin (ICN Biomedical 902111). Colorimetric assays utilizing these substrates are well known in the art (See e.g., WO 99/34011 and US 6,376,450). The pNA peptidyl assay (See e.g., Del Mar et al., Anal Biochem, 99:316-320, 1979) also finds use in determining the active enzyme concentration. This assay measures the rate at which p-nitroaniline is released as the enzyme hydrolyzes a soluble synthetic substrate, such as succinyl-alanine-alanine-proline-phenylalanine-p-nitroanilide (suc-AAPF-pNA). The rate of production of yellow color from the hydrolysis reaction is measured at 410 nm on a spectrophotometer and is proportional to the active enzyme concentration. In addition, absorbance measurements at 280 nanometers (nm) can be used to determine the total protein concentration in a sample of purified protein. The activity on substrate/protein concentration gives the enzyme specific activity.

The term "subtilisin variant" refers to a subtilisin that differs from a reference subtilisin by one or more naturally-occurring or man-made substitutions, insertions, or deletions of an amino acid. In another embodiment, the reference subtilisin can be a naturally occurring and/or recombinant subtilisin protease. In a still further embodiment, the reference subtilisin is a BspE04637-clade subtilisin. In an even still further embodiment, the reference subtilisin is a BspE04637-clade subtilisin comprising a motif selected from Motif 1, Motif 2, Motif 3, Motif 4, Motif 5, Motif 6, and Motif 7. The BspE04637-clade of subtilisins is more fully described in WO2016/069557. In yet another embodiment, the reference subtilisin comprises an amino acid sequence having 90%, 95%, or 96% amino acid sequence identity to the amino acid sequence of SEQ ID NO:1 or 34.

As used herein, "the genus Bacillus" includes all species within the genus "Bacillus," as known to those of skill in the art, including but not limited to *B. subtilis, B. licheniformis, B. lentus, B. brevis, B. stearothermophilus, B. alkalophilus, B. amyloliquefaciens, B. clausii, B. halodurans, B. megaterium, B. coagulans, B. circulans, B. gibsonii,* and *B. thuringiensis.* It is recognized that the genus *Bacillus* continues to undergo taxonomical reorganization. Thus, it is intended that the genus include species that have been reclassified, including but not limited to such organisms as *B. stearothermophilus*, which is now named "*Geobacillus stearothermophilus*", or *B. polymyxa,* which is now *"Paenibacillus polymyxa".* The production of resistant endospores under stressful environmental conditions is considered the defining feature of the genus *Bacillus,* although this characteristic also applies to the recently named *Alicyclobacillus, Amphibacillus, Aneurinibacillus, Anoxybacillus, Brevibacillus, Filobacillus, Gracilibacillus, Halobacillus, Paenibacillus, Salibacillus, Thermobacillus, Ureibacillus,* and *Virgibacillus.*

As used herein, the term "mutation" refers to changes made to a reference amino acid or nucleic acid sequence. It is intended that the term encompass substitutions, insertions and deletions.

As used herein, the term "vector" refers to a nucleic acid construct used to introduce or transfer nucleic acid(s) into a target cell or tissue. A vector is typically used to introduce foreign DNA into a cell or tissue. Vectors include plasmids, cloning vectors, bacteriophages, viruses (e.g., viral vector), cosmids, expression vectors, shuttle vectors, and the like. A vector typically includes an origin of replication, a multicloning site, and a selectable marker. The process of inserting a vector into a target cell is typically referred to as transformation. The present invention includes, in some embodiments, a vector that comprises a DNA sequence encoding a serine protease polypeptide (e.g., precursor or mature serine protease polypeptide) that is operably linked to a suitable prosequence (e.g., secretory, signal peptide sequence, etc.) capable of effecting the expression of the DNA sequence in a suitable host, and the folding and translocation of the recombinant polypeptide chain.

As used herein in the context of introducing a nucleic acid sequence into a cell, the term "introduced" refers to any method suitable for transferring the nucleic acid sequence into the cell. Such methods for introduction include but are not limited to protoplast fusion, transfection, transformation, electroporation, conjugation, and transduction. Transformation refers to the genetic alteration of a cell which results from the uptake, optional genomic incorporation, and expression of genetic material (e.g., DNA).

"Expression cassette" or "expression vector" refers to a nucleic acid construct or vector generated recombinantly or synthetically for the expression of a nucleic acid of interest (e.g., a foreign nucleic acid or transgene) in a target cell. The nucleic acid of interest typically expresses a protein of interest. An expression vector or expression cassette typically comprises a promoter nucleotide sequence that drives or promotes expression of the foreign nucleic acid. The expression vector or cassette also typically includes other specified nucleic acid elements that permit transcription of a particular nucleic acid in a target cell. A recombinant expression cassette can be incorporated into a plasmid, chromosome, mitochondrial DNA, plastid DNA, virus, or nucleic acid fragment. Some expression vectors have the ability to incorporate and express heterologous DNA fragments in a host cell or genome of the host cell. Many prokaryotic and eukaryotic expression vectors are commercially available. Selection of appropriate expression vectors for expression of a protein from a nucleic acid sequence incorporated into the expression vector is within the knowledge of those of skill in the art.

As used herein, a nucleic acid is "operably linked" with another nucleic acid sequence when it is placed into a functional relationship with another nucleic acid sequence. For example, a promoter or enhancer is operably linked to a nucleotide coding sequence if the promoter affects the transcription of the coding sequence. A ribosome binding site may be operably linked to a coding sequence if it is positioned so as to facilitate translation of the coding sequence. Typically, "operably linked" DNA sequences are contiguous. However, enhancers do not have to be contiguous. Linking is accomplished by ligation at convenient restriction sites. If such sites do not exist, synthetic oligonucleotide adaptors or linkers may be used in accordance with conventional practice.

As used herein the term "gene" refers to a polynucleotide (e.g., a DNA segment), that encodes a polypeptide and includes regions preceding and following the coding regions. In some instances, a gene includes intervening sequences (introns) between individual coding segments (exons).

As used herein, "recombinant" when used with reference to a cell typically indicates that the cell has been modified by the introduction of a foreign nucleic acid sequence or that the cell is derived from a cell so modified. For example, a recombinant cell may comprise a gene not found in identical form within the native (non-recombinant) form of the cell, or a recombinant cell may comprise a native gene (found in the native form of the cell) that has been modified and re-introduced into the cell. A recombinant cell may comprise a nucleic acid endogenous to the cell that has been modified without removing the nucleic acid from the cell; such modifications include those obtained by gene replacement, site-specific mutation, and related techniques known to those of ordinary skill in the art. Recombinant DNA technology includes techniques for the production of recombinant DNA in vitro and transfer of the recombinant DNA into cells where it may be expressed or propagated, thereby producing a recombinant polypeptide. "Recombination" and "recombining" of polynucleotides or nucleic acids refer generally to the assembly or combining of two or more nucleic acid or polynucleotide strands or fragments to generate a new polynucleotide or nucleic acid.

A nucleic acid or polynucleotide is said to "encode" a polypeptide if, in its native state or when manipulated by methods known to those of skill in the art, it can be transcribed and/or translated to produce the polypeptide or a fragment thereof. The anti-sense strand of such a nucleic acid is also said to encode the sequence.

The terms "host strain" and "host cell" refer to a suitable host for an expression vector comprising a DNA sequence of interest.

A "protein" or "polypeptide" comprises a polymeric sequence of amino acid residues. The terms "protein" and "polypeptide" are used interchangeably herein. The single and 3-letter code for amino acids as defined in conformity with the IUPAC-IUB Joint Commission on Biochemical Nomenclature (JCBN) is used throughout this disclosure. The single letter X refers to any of the twenty amino acids. It is also understood that a polypeptide may be coded for by more than one nucleotide sequence due to the degeneracy of the genetic code.

A "prosequence" or "propeptide sequence" refers to an amino acid sequence between the signal peptide sequence and mature protease sequence that is necessary for the proper folding and secretion of the protease; they are sometimes referred to as intramolecular chaperones. Cleavage of the prosequence or propeptide sequence results in a mature active protease. Bacterial serine proteases are often expressed as pro-enzymes.

The terms "signal sequence" and "signal peptide" refer to a sequence of amino acid residues that may participate in the secretion or direct transport of the mature or precursor form of a protein. The signal sequence is typically located N-terminal to the precursor or mature protein sequence. The signal sequence may be endogenous or exogenous. A signal sequence is normally absent from the mature protein. A signal sequence is typically cleaved from the protein by a signal peptidase after the protein is transported.

The term "mature" form of a protein, polypeptide, or peptide refers to the functional form of the protein, polypeptide, or peptide without the signal peptide sequence and propeptide sequence.

The term "precursor" form of a protein or peptide refers to a mature form of the protein having a prosequence operably linked to the amino or carbonyl terminus of the protein. The precursor may also have a "signal" sequence operably linked to the amino terminus of the prosequence. The precursor may also have additional polypeptides that are involved in post-translational activity (e.g., polypeptides cleaved therefrom to leave the mature form of a protein or peptide).

The term "wild-type" in reference to an amino acid sequence or nucleic acid sequence indicates that the amino acid sequence or nucleic acid sequence is a native or naturally-occurring sequence. As used herein, the term "naturally-occurring" refers to anything (e.g., proteins, amino acids, or nucleic acid sequences) that is found in nature. Conversely, the term "non-naturally occurring" refers to anything that is not found in nature (e.g., recombinant nucleic acids and protein sequences produced in the laboratory or modification of the wild-type sequence).

As used herein with regard to amino acid residue positions, "corresponding to" or "corresponds to" or "corresponds" refers to an amino acid residue at the enumerated position in a protein or peptide, or an amino acid residue that is analogous, homologous, or equivalent to an enumerated residue in a protein or peptide. As used herein, "corresponding region" generally refers to an analogous position in a related proteins or a reference protein.

The terms "derived from" and "obtained from" refer to not only a protein produced or producible by a strain of the organism in question, but also a protein encoded by a DNA sequence isolated from such strain and produced in a host organism containing such DNA sequence. Additionally, the term refers to a protein which is encoded by a DNA sequence of synthetic and/or cDNA origin and which has the identifying characteristics of the protein in question. To exemplify, "proteases derived from Bacillus" refers to those enzymes having proteolytic activity that are naturally produced by Bacillus, as well as to serine proteases like those produced by Bacillus sources but which through the use of genetic engineering techniques are produced by other host cells transformed with a nucleic acid encoding the serine proteases.

The term "identical" in the context of two polynucleotide or polypeptide sequences refers to the nucleic acids or amino acids in the two sequences that are the same when aligned for maximum correspondence, as measured using sequence comparison or analysis algorithms described below and known in the art.

"% identity" or percent identity" or "PID" refers to protein sequence identity. Percent identity may be determined using standard techniques known in the art. The percent amino acid identity shared by sequences of interest can be determined by aligning the sequences to directly compare the sequence information, e.g., by using a program such as BLAST, MUSCLE, or CLUSTAL. The BLAST algorithm is described, for example, in Altschul et al., J Mol Biol, 215:403-410 (1990) and Karlin et al., Proc Natl Acad Sci USA, 90:5873-5787 (1993). A percent (%) amino acid sequence identity value is determined by the number of matching identical residues divided by the total number of residues of the "reference" sequence including any gaps created by the program for optimal/maximum alignment. BLAST algorithms refer to the "reference" sequence as the "query" sequence.

As used herein, "homologous proteins" or "homologous proteases" refers to proteins that have distinct similarity in primary, secondary, and/or tertiary structure. Protein homology can refer to the similarity in linear amino acid sequence when proteins are aligned. Homology can be determined by amino acid sequence alignment, e.g., using a program such as BLAST, MUSCLE, or CLUSTAL. Homologous search of protein sequences can be done using BLASTP and PSI-BLAST from NCBI BLAST with threshold (E-value cut-off) at 0.001. (Altschul et al., "Gapped BLAST and PSI BLAST a new generation of protein database search programs", Nucleic Acids Res, Set 1;25(17):3389-402(1997)). The BLAST program uses several search parameters, most of which are set to the default values. The NCBI BLAST algorithm finds the most relevant sequences in terms of biological similarity but is not recommended for query sequences of less than 20 residues (Altschul et al., Nucleic Acids Res, 25:3389-3402, 1997 and Schaffer et al., Nucleic Acids Res, 29:2994-3005, 2001). Exemplary default BLAST parameters for a nucleic acid sequence searches include: Neighboring words threshold=1 1; E-value cutoff=10; Scoring Matrix=NUC.3.1 (match=1, mismatch=-3); Gap Opening=5; and Gap Extension=2. Exemplary default BLAST parameters for amino acid sequence searches include: Word size = 3; E-value cutoff=10; Scoring Matrix=BLOSUM62; Gap Opening=11; and Gap extension=1. Using this information, protein sequences can be grouped and/or a phylogenetic tree built therefrom. Amino acid sequences can be entered in a program such as the Vector NTI Advance suite and a Guide Tree can be created using the Neighbor Joining (NJ) method (Saitou and Nei, Mol Biol Evol, 4:406-425, 1987). The tree construction can be calculated using Kimura's correction for sequence distance and ignoring positions with gaps. A program such as AlignX can display the calculated distance values in parenthesis following the molecule name displayed on the phylogenetic tree.

Understanding the homology between molecules can reveal the evolutionary history of the molecules as well as information about their function; if a newly sequenced protein is homologous to an already characterized protein, there is a strong indication of the new protein's biochemical function. The most fundamental relationship between two entities is homology; two molecules are said to be homologous if they have been derived from a common ancestor. Homologous molecules, or homologs, can be divided into two classes, paralogs and orthologs. Paralogs are homologs that are present within one species. Paralogs often differ in their detailed biochemical functions. Orthologs are homologs that are present within different species and have very similar or identical functions. A protein superfamily is the largest grouping (clade) of proteins for which common ancestry can be inferred. Usually this common ancestry is based on sequence alignment and mechanistic similarity. Superfamilies typically contain several protein families which show sequence similarity within the family. The term "protein clan" is commonly used for protease superfamilies based on the MEROPS protease classification system.

The CLUSTAL W algorithm is another example of a sequence alignment algorithm (See, Thompson et al., Nucleic Acids Res, 22:4673-4680, 1994). Default parameters for the CLUSTAL W algorithm include: Gap opening penalty=10.0; Gap extension penalty=0.05; Protein weight matrix=BLOSUM series; DNA weight matrix=IUB; Delay divergent sequences %=40; Gap separation distance=8; DNA transitions weight=0.50; List hydrophilic residues=GPSNDQEKR; Use negative matrix=OFF; Toggle Residue specific penalties=ON; Toggle hydrophilic penalties=ON; and Toggle end gap separation penalty=OFF. In CLUSTAL algorithms, deletions occurring at either terminus are included. For example, a variant with a five amino acid deletion at either terminus (or within the polypeptide) of a polypeptide of 500 amino acids would have a percent sequence identity of 99% (495/500 identical residues × 100) relative to the "reference" polypeptide. Such a variant would be encompassed by a variant having "at least 99% sequence identity" to the polypeptide.

A nucleic acid or polynucleotide is "isolated" when it is at least partially or completely separated from other components, including but not limited to for example, other proteins, nucleic acids, cells, etc. Similarly, a polypeptide, protein or peptide is "isolated" when it is at least partially or completely separated from other components, including but not limited to for example, other proteins, nucleic acids, cells, etc. On a molar basis, an isolated species is more abundant than are other species in a composition. For example, an isolated species may comprise at least about 60%, about 65%, about 70%, about 75%, about 80%, about 85%, about 90%, about 91%, about 92%, about 93%, about 94%, about 95%, about 96%, about 97%, about 98%, about 99%, or about 100% (on a molar basis) of all macromolecular species present. Preferably, the species of interest is purified to essential homogeneity (i.e., contaminant species cannot be detected in the composition by conventional detection methods). Purity and homogeneity can be determined using a number of techniques well known in the art, such as agarose or polyacrylamide gel electrophoresis of a nucleic acid or a protein sample, respectively, followed by visualization upon staining. If desired, a high-resolution technique, such as high performance liquid chromatography (HPLC) or a similar means can be utilized for purification of the material.

The term "purified" as applied to nucleic acids or polypeptides generally denotes a nucleic acid or polypeptide that is essentially free from other components as determined by analytical techniques well known in the art (e.g., a purified polypeptide or polynucleotide forms a discrete band in an electrophoretic gel, chromatographic eluate, and/or a media subjected to density gradient centrifugation). For example, a nucleic acid or polypeptide that gives rise to essentially one band in an electrophoretic gel is "purified." A purified nucleic acid or polypeptide is at least about 50% pure, usually at least about 60%, about 65%, about 70%, about 75%, about 80%, about 85%, about 90%, about 91%, about 92%, about 93%, about 94%, about 95%, about 96%, about 97%, about 98%, about 99%, about 99.5%, about 99.6%, about 99.7%, about 99.8% or more pure (e.g., percent by weight on a molar basis). In a related sense, a composition is enriched for a molecule when there is a substantial increase in the concentration of the molecule after application of a purification or enrichment technique. The term "enriched" refers to a compound, polypeptide, cell, nucleic acid, amino acid, or other specified material or component that is present in a composition at a relative or absolute concentration that is higher than a starting composition.

As used herein, the term "functional assay" refers to an assay that provides an indication of a protein's activity. In some embodiments, the term refers to assay systems in which a protein is analyzed for its ability to function in its usual capacity. For example, in the case of a protease, a functional assay involves determining the effectiveness of the protease to hydrolyze a proteinaceous substrate.

The term "cleaning activity" refers to a cleaning performance achieved by a serine protease polypeptide or reference subtilisin under conditions prevailing during the proteolytic, hydrolyzing, cleaning, or other process of the disclosure. In some embodiments, cleaning performance of a serine protease or reference subtilisin may be determined by using various assays for cleaning one or more enzyme sensitive stain on an item or surface (e.g., a stain resulting from food, grass, blood, ink, milk, oil, and/or egg protein). Cleaning performance of one or more subtilisin variant described herein or reference subtilisin can be determined by subjecting the stain on the item or surface to standard wash condition(s) and assessing the degree to which the stain is removed by using various chromatographic, spectrophotometric, or other quantitative methodologies. Exemplary cleaning assays and methods are known in the art and include, but are not limited to those described in WO99/34011 and US 6,605,458, as well as those cleaning assays and methods included in the Examples provided below.

The term "cleaning effective amount" of one or more subtilisin variant described herein or reference subtilisin refers to the amount of protease that achieves a desired level of enzymatic activity in a specific cleaning composition. Such effective amounts are readily ascertained by one of ordinary skill in the art and are based on many factors, such as the particular protease used, the cleaning application, the specific composition of the cleaning composition, and whether a liquid or dry (e.g., granular, tablet, bar) composition is required, etc.

The term "cleaning adjunct material" refers to any liquid, solid, or gaseous material included in cleaning composition other than one or more subtilisin variant described herein. In some embodiments, the cleaning compositions of the present disclosure include one or more cleaning adjunct materials. Each cleaning adjunct material is typically selected depending on the particular type and form of cleaning composition (e.g., liquid, granule, powder, bar, paste, spray, tablet, gel, foam, or other composition). Preferably, each cleaning adjunct material is compatible with the protease enzyme used in the composition.

Cleaning compositions and cleaning formulations include any composition that is suited for cleaning, bleaching, disinfecting, and/or sterilizing any object, item, and/or surface. Such compositions and formulations include, but are not limited to for example, liquid and/or solid compositions, including cleaning or detergent compositions (e.g., liquid, tablet, gel, bar, granule, and/or solid laundry cleaning or detergent compositions and fine fabric detergent compositions; hard surface cleaning compositions and formulations, such as for glass, wood, ceramic and metal counter tops and windows; carpet cleaners; oven cleaners; fabric fresheners; fabric softeners; and textile, laundry booster cleaning or detergent compositions, laundry additive cleaning compositions, and laundry pre-spotter cleaning compositions; dishwashing compositions, including hand or manual dishwashing compositions (e.g., "hand" or "manual" dishwashing detergents) and automatic dishwashing compositions (e.g., "automatic dishwashing detergents"). Single dosage unit forms also find use with the present invention, including but not limited to pills, tablets, gelcaps, or other single dosage units such as pre-measured powders or liquids.

Cleaning composition or cleaning formulations, as used herein, include, unless otherwise indicated, granular or powder-form all-purpose or heavy-duty washing agents, especially cleaning detergents; liquid, granular, gel, solid, tablet, paste, or unit dosage form all-purpose washing agents, especially the so-called heavy-duty liquid (HDL) detergent or heavy-duty dry (HDD) detergent types; liquid fine-fabric detergents; hand or manual dishwashing agents, including those of the high-foaming type; hand or manual dishwashing, automatic dishwashing, or dishware or tableware washing agents, including the various tablet, powder, solid, granular, liquid, gel, and rinse-aid types for household and institutional use; liquid cleaning and disinfecting agents, including antibacterial hand-wash types, cleaning bars, mouthwashes, denture cleaners, car shampoos, carpet shampoos, bathroom cleaners; hair shampoos and/or hair-rinses for humans and other animals; shower gels and foam baths and metal cleaners; as well as cleaning auxiliaries, such as bleach additives and "stain-stick" or pre-treat types. In some embodiments, granular compositions are in "compact" form; in some embodiments, liquid compositions are in a "concentrated" form.

As used herein, "fabric cleaning compositions" include hand and machine laundry detergent compositions including laundry additive compositions and compositions suitable for use in the soaking and/or pretreatment of stained fabrics (e.g., clothes, linens, and other textile materials).

As used herein, "non-fabric cleaning compositions" include non-textile (i.e., non-fabric) surface cleaning compositions, including, but not limited to for example, hand or manual or automatic dishwashing detergent compositions, oral cleaning compositions, denture cleaning compositions, contact lens cleaning compositions, wound debridement compositions, and personal cleansing compositions.

As used herein, the term "detergent composition" or "detergent formulation" is used in reference to a composition intended for use in a wash medium for the cleaning of soiled or dirty objects, including particular fabric and/or non-fabric objects or items. In some embodiments, the detergents of the disclosure comprise one or more subtilisin variant described herein and, in addition, one or more surfactants, transferase(s), hydrolytic enzymes, oxido reductases, builders (e.g., a builder salt), bleaching agents, bleach activators, bluing agents, fluorescent dyes, caking inhibitors, masking agents, enzyme activators, antioxidants, and/or solubilizers. In some instances, a builder salt is a mixture of a silicate salt and a phosphate salt, preferably with more silicate (e.g., sodium metasilicate) than phosphate (e.g., sodium tripolyphosphate). Some embodiments are directed to cleaning compositions or detergent compositions that do not contain any phosphate (e.g., phosphate salt or phosphate builder).

As used herein, the term "bleaching" refers to the treatment of a material (e.g., fabric, laundry, pulp, etc.) or surface for a sufficient length of time and/or under appropriate pH and/or temperature conditions to effect a brightening (i.e., whitening) and/or cleaning of the material. Examples of chemicals suitable for bleaching include, but are not limited to, for example, ClO₂, H₂O₂, peracids, NO₂, etc.

As used herein, "wash performance" of a protease (e.g., one or more subtilisin variant described herein) refers to the contribution of one or more subtilisin variant described herein to washing that provides additional cleaning performance to the detergent as compared to the detergent without the addition of the one or more subtilisin variant described herein to the composition. Wash performance is compared under relevant washing conditions. In some test systems, other relevant factors, such as detergent composition, sud concentration, water hardness, washing mechanics, time, pH, and/or temperature, can be controlled in such a way that condition(s) typical for household application in a certain market segment (e.g., hand or manual dishwashing, automatic dishwashing, dishware cleaning, tableware cleaning, fabric cleaning, etc.) are imitated.

The term "relevant washing conditions" is used herein to indicate the conditions, particularly washing temperature, time, washing mechanics, sud concentration, type of detergent and water hardness, actually used in households in a hand dishwashing, automatic dishwashing, or laundry detergent market segment.

As used herein, the term "disinfecting" refers to the removal of contaminants from the surfaces, as well as the inhibition or killing of microbes on the surfaces of items.

The "compact" form of the cleaning compositions herein is best reflected by density and, in terms of composition, by the amount of inorganic filler salt. Inorganic filler salts are conventional ingredients of detergent compositions in powder form. In conventional detergent compositions, the filler salts are present in substantial amounts, typically about 17 to about 35% by weight of the total composition. In contrast, in compact compositions, the filler salt is present in amounts not exceeding about 15% of the total composition. In some embodiments, the filler salt is present in amounts that do not exceed about 10%, or more preferably, about 5%, by weight of the composition. In some embodiments, the inorganic filler salts are selected from the alkali and alkaline-earth-metal salts of sulfates and chlorides. In some embodiments, the filler salt is sodium sulfate.

Disclosed herein is one or more subtilisin variant useful for cleaning applications and in methods of cleaning, as well as in a variety of industrial applications. Disclosed herein is one or more isolated, recombinant, substantially pure, or non-naturally occurring subtilisin variant. The subtilisin variants of the invention are as set out in the claims. In some embodiments, the one or more subtilisin variant described herein is useful in cleaning applications and can be incorporated into cleaning compositions that are useful in methods of cleaning an item or a surface in need thereof.

The invention provides a subtilisin variant comprising an amino acid sequence having at least 90% sequence identity to the amino acid sequence of SEQ ID NO: 1 or 34 and comprising an amino acid substitution X 120R wherein the amino acid positions of the variant are numbered by correspondence with the amino acid sequence of SEQ ID NO: 1, and wherein said variant has protease activity, wherein said variant has improved cleaning performance when compared to a reference subtilisin comprising an amino acid sequence of SEQ ID NO: 1 or 34.

The variant may comprise an amino acid sequence additionally comprising one, two, three, or four or more amino acid substitutions selected from X12K, X14D, X15K, X26R, X37A, X48R, X53G/R, X55R, X56K, X78D, X101R, X105K, X122H, X133R, X143R, X155V, X164N, X165R, X166N, X169D, X175N/P/S, X184S, X197K, X198E, X220R, X238K, X243R, X248Q, X254K, X255N, X259R, X264K, X268K, X272N, X274E, and X27551K wherein X is any amino acid.

In some embodiments (i) the amino acid substitution at position 197 is not an S; (ii) amino acid substitution at position 238 is not a W; (iii) amino acid substitution at position 268 is not H; (iv) said variant does not consist of the amino acid sequence of BAD02409 or JP2003325186-0001; and/or (v) said variant does not consist of the amino acid sequence of SWT183_1430046.

In some embodiments the subtilisin variant comprises an amino acid sequence comprising one, two, three, or four or more amino acid substitutions selected from (i) Q12K, T14D, D15K, V26R, R37A, H48R, D53G/R, A55R, N56K, N78D, S101R, S105K, G/N120R, D122H, S133R, I143R, A155V, R164N, G165R, D166N, G169D, D175N/P/S, D184S, P/S197K, A198E, T220R, L/W238K, N243R, E248Q, N254K, D255N, N259R, N264K, H/N268K, R272N, V274E, and D275S/K; or (ii) Q12K, T14D, D15K, V26R, R37A, H48R, D53G/R, A55R, N56K, N78D, S101R, S105K, N120R, D122H, S133R, I143R, A155V, R164N, G165R, D166N, G169D, D175N/P/S, D184S, P197K, A198E, T220R, L238K, N243R, E248Q, N254K, D255N, N259R, N264K, N268K, R272N, V274E, and D275S/K; and wherein the amino acid positions of the variant are numbered by correspondence with the amino acid sequence of SEQ ID NO: 1.

A still further embodiment is directed to an subtilisin variant comprising an amino acid sequence comprising nine or more amino acid substitutions selected from: R37A-D53G-S101R-N120R-I143R-D166N-D184S-P197K-L238K-E248Q-D255N-R272N; D15K-A55R-S101R-N120R-I143R-D166N-D184S-P197K-L238K-E248Q-N268K-V274E; H48R-A55R-S101R-N120R-S133R-R164N-L238K-E248Q-D255N-N268K; D15K-H48R-A55R-S101R-N120R-I143R-G165R-D175N-A198E-N243R-N254K-D275K; D15K-D53R-S101R-N120R-I143R-G165R-D175S-N243R-D255N-N264K-D275K; and combinations thereof; and wherein the amino acid positions of the variant are numbered by correspondence with the amino acid sequence of SEQ ID NO: 1.

In some embodiments, the one or more subtilisin variant described herein is a BspE04637-clade subtilisin. The BspE04637-clade of subtilisins is characterized by a 2 amino acid residue insertion after position N56, wherein the amino acid position is numbered by correspondence with the amino acid sequence of subtilisin BPN'. This insertion occurs in the span of residues linking the catalytic aspartic acid (D32) and catalytic histidine (H66) that form part of the characteristic catalytic triad, wherein the amino acid positions are numbered by correspondence with the amino acid sequence of subtilisin BspE04637. The BspE04637-clade subtilisins are more fully described in WO2016/069557.

In some embodiments, the one or more subtilisin variant described herein comprises a DTGIXXXHXDLXXXGGXSVFXXX XXXXXXXDXXGH (SEQ ID NO:35) motif, wherein the initial D is the active site Aspartic acid, the terminal H is the active site Histidine, and X is any amino acid ("Motif 1"). In other embodiments the one or more subtilisin variant described herein comprises a DTGI XXXHXDLXXXGGXSVFXXXXXXDPXXDXXGH (SEQ ID NO:36) motif, wherein the initial D is the active site Aspartic acid, the terminal H is the active site Histidine, and X is any amino acid ("Motif 2"). In still further embodiments, the one or more subtilisin variant described herein comprises a DTGIXXXH XDLNVXGGXSVFXXXXXXXXXXDXXGH (SEQ ID NO:37) motif, wherein the initial D is the active site Aspartic acid, the terminal H is the active site Histidine, and X is any amino acid ("Motif 3"). In yet another embodiment, the one or more subtilisin variant described herein comprises a DTGIXXXHXDLN VXGGXSVFXXXXXXDPXXDXXGH (SEQ ID NO:38) motif, wherein the initial D is the active site Aspartic acid, the terminal H is the active site Histidine, and X is any amino acid ("Motif 4"). In some embodiments, the one or more subtilisin variant described herein comprises a DTGIDXXHXDLNVXGG XSVFXXXXXXXXXXDXXGH (SEQ ID NO:39) motif, wherein the initial D is the active site Aspartic acid and the terminal H is the active site Histidine, and X is any amino acid ("Motif 5"). In some embodiments, the one or more subtilisin variant described herein comprises a DTGIDXNHXDLNVRGG XSVFTXXXXXDPXXDXXGH (SEQ ID NO:40) motif, wherein the initial D is the active site Aspartic acid, the terminal H is the active site Histidine, and X is any amino acid ("Motif 6"). In some embodiments, the one or more subtilisin variant described herein comprises a DTGIDXNHXDLNVRGG XSVFTXXXXXDPYYDXXGH (SEQ ID NO:41) motif, wherein the initial D is the active site Aspartic acid and the terminal H is the active site Histidine, and X is any amino acid ("Motif 7").

In some embodiments, the one or more subtilisin variant described herein comprises a DTGIXXXHXDLXXXGGXSVFXXXXXXXXXXDXXGH (SEQ ID NO:35) motif, wherein the initial D is the active site Aspartic acid, the terminal H is the active site Histidine, and X is any amino acid, with the proviso that the variant does not consist of the amino acid sequence of BAD02409 or JP2003325186-0001. In other embodiments, the one or more subtilisin variant described herein comprises a DTGIXXX HXDLXXXGGXSVFXXXXXXDPXXDXXGH (SEQ ID NO:36) motif, wherein the initial D is the active site Aspartic acid, the terminal H is the active site Histidine, and X is any amino acid, with the proviso that the variant does not consist of the amino acid sequence of BAD02409 or JP2003325186-0001. In still further embodiments, the one or more subtilisin variant described herein comprises a DTGI XXXHXDLNVXGGXSVFXXXXXX XXXXDXXGH (SEQ ID NO:37) motif, wherein the initial D is the active site Aspartic acid, the terminal H is the active site Histidine, and X is any amino acid, with the proviso that the variant does not consist of the amino acid sequence of BAD02409 or JP2003325186-0001. In yet another embodiment, the one or more subtilisin variant described herein comprises a DTGIX XXHXDLNVXGGXSVFXXXXXXDPXXDXXGH (SEQ ID NO:38) motif, wherein the initial D is the active site Aspartic acid, the terminal H is the active site Histidine, and X is any amino acid, with the proviso that the variant does not consist of the amino acid sequence of BAD02409 or JP2003325186-0001. In some embodiments, the one or more subtilisin variant described herein comprises a DTGIDXX HXDLNVXGGXSVFXXXXXX XXXXDXXGH (SEQ ID NO:39) motif, wherein the initial D is the active site Aspartic acid and the terminal H is the active site Histidine, and X is any amino acid, with the proviso that the variant does not consist of the amino acid sequence of BAD02409 or JP2003325186-0001. In some embodiments, the one or more subtilisin variant described herein comprises a DTGIDXN HXDLNVRGGXSVFTXXXXXDPXXDXXGH (SEQ ID NO:40) motif, wherein the initial D is the active site Aspartic acid, the terminal H is the active site Histidine, and X is any amino acid, with the proviso that the one or more subtilisin variant described herein does not consist of the amino acid sequence of BAD02409 or JP2003325186-0001. In some embodiments, the one or more subtilisin variant described herein comprises a DTGIDXNHXDLNVRGGXSVFTXXXXXDPYYDXXGH (SEQ ID NO:41) motif, wherein the initial D is the active site Aspartic acid and the terminal H is the active site Histidine, and X is any amino acid, with the proviso that the variant does not consist of the amino acid sequence of BAD02409 or JP2003325186-0001.

In some embodiments, one or more subtilisin variant described herein comprises an amino acid sequence having 90%, 95% or 96%, amino acid sequence identity to the amino acid sequence of SEQ ID NO:1 or 34. In some embodiments, the one or more subtilisin variant described herein comprises an amino acid sequence having at least 90%, or 95% amino acid sequence identity to the amino acid sequence of SEQ ID NO: 1 or 34. In some embodiments, the one or more subtilisin variant described herein comprises an amino acid sequence having at least 90%, or 95% amino acid sequence identity to the amino acid sequence of SEQ ID NO: 1 or 34, with the proviso that said variant does not consist of the amino acid sequence of BAD02409 or JP2003325186-0001. Homology can be determined by amino acid sequence alignment, e.g., using a program such as BLAST, MUSCLE, ALIGN, or CLUSTAL, as described herein. In some embodiments, the one or more subtilisin variant described herein is an isolated, recombinant, substantially pure, or non-naturally occurring subtilisin having subtilisin activity or casein hydrolysis activity (for example, dimethylcasein hydrolysis activity).

As noted above, the one or more subtilisin variant described herein has enzymatic activity (e.g., protease activity) and thus is useful in cleaning applications, including but not limited to, methods for cleaning dishware items, tableware items, fabrics, and items having hard surfaces (e.g., the hard surface of a table, table top, wall, furniture item, floor, ceiling, etc.). Exemplary cleaning compositions comprising one or more subtilisin variant described herein are described infra. The enzymatic activity (e.g., protease enzyme activity) of one or more subtilisin variant described herein can be determined readily using procedures well known to those of ordinary skill in the art. The Examples presented infra describe methods for evaluating the enzymatic activity and cleaning performance. The performance of polypeptide enzymes of the invention in removing stains (e.g., a protein stain such as blood/milk/ink, pigment/milk/ink or egg yolk), cleaning hard surfaces, or cleaning laundry, dishware or tableware item(s) can be readily determined using procedures well known in the art and/or by using procedures set forth in the Examples.

The subtilisin variants of the invention have improved cleaning performance when compared to a reference subtilisin comprising an amino acid sequence of SEQ ID NO: 1 or 34, wherein said variant has a BMI, POM, and/or egg yolk with pigment swatch cleaning PI >1 wherein said cleaning performance in detergent is measured in accordance the cleaning performance in detergent assay of Example 1. In some embodiments, the one or more subtilisin variant described herein has one or more additional improved property when compared to a reference subtilisin, wherein the improved property is selected from improved protease activity and improved thermostability in detergent, wherein the reference subtilisin comprises an amino acid sequence of SEQ ID NO: 1 or 34. In one embodiment, the subtilisin variant described herein is more stable through a longer wash period as compared to a reference subtilisin. In another embodiment, the subtilisin variant described herein is more stable through a short and cool or long and hot wash-cycle as compared to a reference subtilisin. In a still yet further embodiment, the one or more improved property is (i) improved protease activity, wherein said variant has a PI > 1 on N-suc-AAPF-pNA or dimethyl casein substrate; and/or (ii) improved thermostability, wherein said variant has a stability PI > 1. In an even further embodiment, one or more subtilisin variant described herein has improved protease activity, wherein said variant has a 1 on N-suc-AAPF-pNA or dimethyl casein substrate. In another embodiment, one or more subtilisin variant described herein has improved thermostability, wherein said variant has a stability PI > 1. In another embodiment, one or more subtilisin variant described herein has improved protease activity, wherein said variant has a PI > 1 on N-suc-AAPF-pNA or dimethyl casein substrate and said PI is measured in accordance with the protease activity assay of Example 1. In an even further embodiment, one or more subtilisin variant described herein has improved thermostability, wherein said variant has a stability PI > 1 and said PI is measured in accordance with the thermostability in detergent assay of Example 1.

The subtilisin variants of the invention demonstrate cleaning performance in a cleaning composition. Cleaning compositions often include ingredients harmful to the stability and performance of enzymes, making cleaning compositions a harsh environment for enzymes, e.g. serine proteases, to retain function. Thus, it is not trivial for an enzyme to be put in a cleaning composition and expect enzymatic function (e.g. serine protease activity, such as demonstrated by cleaning performance). In some embodiments, the one or more subtilisin variant described herein demonstrates cleaning performance in automatic dishwashing (ADW) detergent compositions. In some embodiments, the cleaning performance in ADW detergent compositions includes cleaning of egg yolk stains. In some embodiments, the one or more subtilisin variant described herein demonstrates cleaning performance in laundry detergent compositions. In some embodiments, the cleaning performance in laundry detergent compositions includes cleaning of blood/milk/ink stains. In each of the cleaning compositions, the one or more subtilisin variant described herein demonstrates cleaning performance with or without a bleach component.

One or more subtilisin variant described herein can be subject to various changes, such as one or more amino acid insertions, deletions, and/or substitutions, either conservative or non-conservative, including where such changes do not substantially alter the enzymatic activity of the variant. Similarly, a nucleic acid of the invention can also be subject to various changes, such as one or more substitutions of one or more nucleotides in one or more codons such that a particular codon encodes the same or a different amino acid, resulting in either a silent variation (e.g., when the encoded amino acid is not altered by the nucleotide mutation) or non-silent variation, one or more deletions of one or more nucleic acids (or codons) in the sequence, one or more additions or insertions of one or more nucleic acids (or codons) in the sequence, and/or cleavage of or one or more truncations of one or more nucleic acids (or codons) in the sequence. Many such changes in the nucleic acid sequence may not substantially alter the enzymatic activity of the resulting encoded polypeptide enzyme compared to the polypeptide enzyme encoded by the original nucleic acid sequence. A nucleic acid sequence described herein can also be modified to include one or more codons that provide for optimum expression in an expression system (e.g., bacterial expression system), while, if desired, said one or more codons still encode the same amino acid(s).

The invention provides polynucleotides as set out in the claims. Described herein is one or more isolated, non-naturally occurring, or recombinant polynucleotide comprising a nucleic acid sequence that encodes one or more subtilisin variant described herein. One or more nucleic acid sequence described herein is useful in recombinant production (e.g., expression) of one or more subtilisin variant described herein, typically through expression of a plasmid expression vector comprising a sequence encoding the one or more subtilisin variant described herein or fragment thereof. The invention provides nucleic acids encoding one or more subtilisin variant of the invention, wherein the variant is a mature form having proteolytic activity. In some embodiments, the one or more subtilisin variant described herein is expressed recombinantly with a homologous pro-peptide sequence. In other embodiments, the one or more subtilisin variant described herein is expressed recombinantly with a heterologous pro-peptide sequence (e.g., GG36 pro-peptide sequence).

One or more nucleic acid sequence described herein can be generated by using any suitable synthesis, manipulation, and/or isolation techniques, or combinations thereof. For example, one or more polynucleotide described herein may be produced using standard nucleic acid synthesis techniques, such as solid-phase synthesis techniques that are well-known to those skilled in the art. In such techniques, fragments of up to 50 or more nucleotide bases are typically synthesized, then joined (e.g., by enzymatic or chemical ligation methods) to form essentially any desired continuous nucleic acid sequence. The synthesis of the one or more polynucleotide described herein can be also facilitated by any suitable method known in the art, including but not limited to chemical synthesis using the classical phosphoramidite method *(See e.g.,* Beaucage et al. Tetrahedron Letters 22:1859-69 (1981)), or the method described in Matthes et al., EMBO J. 3:801-805 (1984) as is typically practiced in automated synthetic methods. One or more polynucleotide described herein can also be produced by using an automatic DNA synthesizer. Customized nucleic acids can be ordered from a variety of commercial sources (e.g., Midland Certified Reagent Company, Great American Gene Company, Operon Technologies Inc., and DNA 2.0). Other techniques for synthesizing nucleic acids and related principles are described by, for example, Itakura et al., Ann. Rev. Biochem. 53:323 (1984) and Itakura et al., Science 198:1056 (1984).

Recombinant DNA techniques useful in modification of nucleic acids are well known in the art, such as, for example, restriction endonuclease digestion, ligation, reverse transcription and cDNA production, and polymerase chain reaction (e.g., PCR). One or more polynucleotide described herein may also be obtained by screening cDNA libraries using one or more oligonucleotide probes that can hybridize to or PCR-amplify polynucleotides which encode one or more subtilisin variant described herein. Procedures for screening and isolating cDNA clones and PCR amplification procedures are well known to those of skill in the art and described in standard references known to those skilled in the art. One or more polynucleotide described herein can be obtained by altering a naturally occurring polynucleotide backbone (e.g., that encodes one or more subtilisin variant described herein or reference subtilisin) by, for example, a known mutagenesis procedure (e.g., site-directed mutagenesis, site saturation mutagenesis, and in vitro recombination). A variety of methods are known in the art that are suitable for generating modified polynucleotides described herein that encode one or more subtilisin variant described herein, including, but not limited to, for example, site-saturation mutagenesis, scanning mutagenesis, insertional mutagenesis, deletion mutagenesis, random mutagenesis, site-directed mutagenesis, and directed-evolution, as well as various other recombinatorial approaches.

The invention also provides one or more vector comprising one or more subtilisin variant as set out in the claims (e.g., a polynucleotide encoding one or more subtilisin variant as set out in the claims); expression vectors or expression cassettes comprising one or more nucleic acid or polynucleotide sequence as set out in the claims; isolated, substantially pure, or recombinant DNA constructs comprising one or more nucleic acid or polynucleotide sequence as set out in the claims; and isolated or recombinant cells comprising one or more polynucleotide sequence as set out in the claims.

Some embodiments are directed to one or more recombinant cell comprising one or more vector (e.g., expression vector or DNA construct) as set out in the claims which comprises one or more nucleic acid or polynucleotide sequence as set out in the claims. Some such recombinant cells are transformed or transfected with such at least one vector, although other methods are available and known in the art. Such cells are typically referred to as host cells. Some such cells comprise bacterial cells, including, but not limited to *Bacillus sp.* cells, such as *B. subtilis* cells. Also disclosed herein are recombinant cells (e.g., recombinant host cells) comprising one or more subtilisin described herein.

In some embodiments, one or more vector described herein is an expression vector or expression cassette comprising one or more polynucleotide sequence described herein operably linked to one or more additional nucleic acid segments required for efficient gene expression (e.g., a promoter operably linked to one or more polynucleotide sequence described herein). A vector may include a transcription terminator and/or a selection gene (e.g., an antibiotic resistant gene) that enables continuous cultural maintenance of plasmid-infected host cells by growth in antimicrobial-containing media.

An expression vector may be derived from plasmid or viral DNA, or in alternative embodiments, contains elements of both. Exemplary vectors include, but are not limited to pC194, pJH101, pE194, pHP13 (See, Harwood and Cutting [eds.], Chapter 3, Molecular Biological Methods for Bacillus, John Wiley & Sons (1990); suitable replicating plasmids for *B. subtilis* include those listed on p. 92). (*See also,* Perego, "Integrational Vectors for Genetic Manipulations in Bacillus subtilis"; Sonenshein et al., [eds.]; "Bacillus subtilis and Other Gram-Positive Bacteria: Biochemistry, Physiology and Molecular Genetics", American Society for Microbiology, Washington, D.C. (1993), pp. 615-624); and p2JM103BBI).

For expression and production of a protein of interest (e.g., one or more subtilisin variant described herein) in a cell, one or more expression vector comprising one or more copy of a polynucleotide encoding one or more subtilisin variant described herein, and in some instances comprising multiple copies, is transformed into the cell under conditions suitable for expression of the variant. In some embodiments, a polynucleotide sequence encoding the one or more subtilisin variant as set out in the claims (as well as other sequences included in the vector) is integrated into the genome of the host cell, while in other embodiments, a plasmid vector comprising a polynucleotide sequence encoding the one or more subtilisin variant set out in the claims remains as autonomous extra-chromosomal element within the cell. Some embodiments provide both extrachromosomal nucleic acid elements as well as incoming nucleotide sequences that are integrated into the host cell genome. The vectors described herein are useful for production of the one or more subtilisin variant described herein. In some embodiments, a polynucleotide construct encoding the one or more subtilisin variant as set out in the claims is present on an integrating vector that enables the integration and optionally the amplification of the polynucleotide encoding the variant into the host chromosome. Examples of sites for integration are well known to those skilled in the art. In some embodiments, transcription of a polynucleotide encoding one or more subtilisin variant as set out in claims is effectuated by a promoter that is the wild-type promoter for the selected reference subtilisin. In some other embodiments, the promoter is heterologous to the reference subtilisin, but is functional in the host cell. Exemplary promoters for use in bacterial host cells include, but are not limited to the amyE, amyQ, amyL, pstS, sacB, pSPAC, pAprE, pVeg, pHpaII promoters; the promoter of the *B. stearothermophilus* maltogenic amylase gene; the *B. amyloliquefaciens* (BAN) amylase gene; the *B. subtilis* alkaline protease gene; the *B. clausii* alkaline protease gene; the *B. pumilis* xylosidase gene; the *B. thuringiensis* cryIIIA; and the *B. licheniformis* alpha-amylase gene. Additional promoters include, but are not limited to the A4 promoter, as well as phage Lambda PR or PL promoters and the *E*. *coli* lac, trp or tac promoters.

One or more subtilisin variant described herein can be produced in host cells of any suitable microorganism, including bacteria and fungi. In some embodiments, one or more subtilisin variant as set out in the claims can be produced in Gram-positive bacteria. In some embodiments, the host cells are *Bacillus spp., Streptomyces spp., Escherichia spp., Aspergillus spp., Trichoderma spp., Pseudomonas spp., Corynebacterium spp., Saccharomyces spp.,* or *Pichia spp.* In some embodiments, one or more subtilisin variant as set out in the claims is produced by *Bacillus sp.* host cells. Examples of *Bacillus sp.* host cells that find use in the production of the one or more subtilisin variant as set out in the claims include, but are not limited to *B. licheniformis, B. lentus, B. subtilis, B. amyloliquefaciens, B. lentus, B. brevis, B. stearothermophilus, B. alkalophilus, B. coagulans, B. circulans, B. pumilis, B. thuringiensis, B. clausii,* and *B. megaterium,* as well as other organisms within the genus *Bacillus.* In some embodiments, *B. subtilis* host cells are used for production of serine protease polypeptides. US 5,264,366 and 4,760,025 (RE 34,606) describe various *Bacillus* host strains that can be used for producing one or more subtilisin variant described herein, although other suitable strains can be used.

Several bacterial strains that can be used to produce one or more subtilisin variant described herein include non-recombinant (i.e., wild-type) *Bacillus sp.* strains, as well as variants of naturally-occurring strains and/or recombinant strains. In some embodiments, the host strain is a recombinant strain, wherein a polynucleotide encoding one or more subtilisin variant as set out in the claims has been introduced into the host. In some embodiments, the host strain is a *B. subtilis* host strain and particularly a recombinant *B. subtilis* host strain. Numerous *B. subtilis* strains are known, including, but not limited to for example, 1A6 (ATCC 39085), 168 (1A01), SB19, W23, Ts85, B637, PB1753 through PB1758, PB3360, JH642, 1A243 (ATCC 39,087), ATCC 21332, ATCC 6051, MI113, DE100 (ATCC 39,094), GX4931, PBT 110, and PEP 211strain *(See e.g.,* Hoch et al., Genetics 73:215-228 (1973); *See also,* US 4,450,235; US 4,302,544; and EP 0134048). The use of *B. subtilis* as an expression host cell is well known in the art *(See e.g.,* Palva et al., Gene 19:81-87 (1982); Fahnestock and Fischer, J. Bacteriol., 165:796-804 (1986); and Wang et al., Gene 69:39-47 (1988)).

In some embodiments, the *Bacillus* host cell is a *Bacillus sp.* that includes a mutation or deletion in at least one of the following genes: degU, degS, degR and degQ. In some embodiments, the mutation is in a degU gene, and in some embodiments the mutation is degU(Hy)32 (*See e.g.,* Msadek et al., J. Bacteriol. 172:824-834 (1990); and Olmos et al., Mol. Gen. Genet. 253:562-567 (1997)). In some embodiments, the *Bacillus* host comprises a mutation or deletion in scoC4 (*See e.g.,* Caldwell et al., J. Bacteriol. 183:7329-7340 (2001)); spoIIE (*See e.g.,* Arigoni et al., Mol. Microbiol. 31:1407-1415 (1999)); and/or oppA or other genes of the opp operon (*See e.g.,* Perego et al., Mol. Microbiol. 5: 173-185 (1991)). Indeed, it is contemplated that any mutation in the opp operon that causes the same phenotype as a mutation in the oppA gene will find use in some embodiments of the altered *Bacillus* strain described herein. In some embodiments, these mutations occur alone, while in other embodiments, combinations of mutations are present. In some embodiments, an altered *Bacillus* host cell strain that can be used to produce one or more subtilisin variant as set out in the claims is a *Bacillus* host strain that already includes a mutation in one or more of the above-mentioned genes. In addition, *Bacillus sp.* host cells that comprise mutation(s) and/or deletions of endogenous protease genes find use. In some embodiments, the *Bacillus* host cell comprises a deletion of the aprE and the nprE genes. In other embodiments, the *Bacillus sp.* host cell comprises a deletion of 5 protease genes, while in other embodiments, the *Bacillus sp.* host cell comprises a deletion of 9 protease genes (*See e.g.,* US 2005/0202535).

Host cells are transformed with one or more nucleic acid sequence encoding one or more subtilisin variant as set out in the claims using any suitable method known in the art. Methods for introducing a nucleic acid (e.g., DNA) into *Bacillus* cells or *E*. *coli* cells utilizing plasmid DNA constructs or vectors and transforming such plasmid DNA constructs or vectors into such cells are well known. In some embodiments, the plasmids are subsequently isolated from *E*. *coli* cells and transformed into *Bacillus* cells. However, it is not essential to use intervening microorganisms such as *E*. *coli,* and in some embodiments, a DNA construct or vector is directly introduced into a *Bacillus* host.

Exemplary methods for introducing one or more nucleic acid sequence described herein into *Bacillus* cells are described in, for example, Ferrari et al., "Genetics," in Harwood et al. [eds.], Bacillus, Plenum Publishing Corp. (1989), pp. 57-72; Saunders et al., J. Bacteriol. 157:718-726 (1984); Hoch et al., J. Bacteriol. 93:1925-1937 (1967); Mann et al., Current Microbiol. 13:131-135 (1986); Holubova, Folia Microbiol. 30:97 (1985); Chang et al., Mol. Gen. Genet. 168:11-115 (1979); Vorobjeva et al., FEMS Microbiol. Lett. 7:261-263 (1980); Smith et al., Appl. Env. Microbiol. 51:634 (1986); Fisher et al., Arch. Microbiol. 139:213-217 (1981); and McDonald, J. Gen. Microbiol. 130:203 (1984)). Indeed, such methods as transformation, including protoplast transformation and transfection, transduction, and protoplast fusion are well known and suited for use herein. Methods known in the art to transform *Bacillus* cells include such methods as plasmid marker rescue transformation, which involves the uptake of a donor plasmid by competent cells carrying a partially homologous resident plasmid *(*S*ee,* Contente et al., Plasmid 2:555-571 (1979); Haima et al., Mol. Gen. Genet. 223:185-191 (1990); Weinrauch et al., J. Bacteriol. 154:1077-1087 (1983); and Weinrauch et al., J. Bacteriol. 169:1205-1211 (1987)). In this method, the incoming donor plasmid recombines with the homologous region of the resident "helper" plasmid in a process that mimics chromosomal transformation.

In addition to commonly used methods, in some embodiments, host cells are directly transformed with a DNA construct or vector comprising a nucleic acid encoding one or more subtilisin variant as set out in the claims (i.e., an intermediate cell is not used to amplify, or otherwise process, the DNA construct or vector prior to introduction into the host cell). Introduction of the DNA construct or vector described herein into the host cell includes those physical and chemical methods known in the art to introduce a nucleic acid sequence (e.g., DNA sequence) into a host cell without insertion into the host genome. Such methods include, but are not limited to calcium chloride precipitation, electroporation, naked DNA, and liposomes. In additional embodiments, DNA constructs or vector are co-transformed with a plasmid, without being inserted into the plasmid. In further embodiments, a selective marker is deleted from the altered *Bacillus* strain by methods known in the art (*See,* Stahl et al., J. Bacteriol. 158:411-418 (1984); and Palmeros et al., Gene 247:255 -264 (2000)).

In some embodiments, the transformed cells of the present invention are cultured in conventional nutrient media. The suitable specific culture conditions, such as temperature, pH and the like are known to those skilled in the art and are well described in the scientific literature. Some embodiments provide a culture (e.g., cell culture) comprising one or more subtilisin variant as set out in the claims or one or more nucleic acid sequence as set out in the claims.

In some embodiments, host cells transformed with one or more polynucleotide sequence encoding one or more subtilisin variant as set out in the claims are cultured in a suitable nutrient medium under conditions permitting the expression of the variant, after which the resulting variant is recovered from the culture. In some embodiments, the variant produced by the cells is recovered from the culture medium by conventional procedures, including, but not limited to, for example, separating the host cells from the medium by centrifugation or filtration, precipitating the proteinaceous components of the supernatant or filtrate by means of a salt (e.g., ammonium sulfate), and chromatographic purification (e.g., ion exchange, gel filtration, affinity, etc.).

In some embodiments, the variant produced by a recombinant host cell of the invention is secreted into the culture medium. A nucleic acid sequence that encodes a purification facilitating domain may be used to facilitate purification of the variant. A vector or DNA construct comprising a polynucleotide sequence encoding one or more subtilisin variant described herein may further comprise a nucleic acid sequence encoding a purification facilitating domain to facilitate purification of the variant (*See e.g.,* Kroll et al., DNA Cell Biol. 12:441-53 (1993)). Such purification facilitating domains include, but are not limited to, for example, metal chelating peptides such as histidine-tryptophan modules that allow purification on immobilized metals (See, Porath, Protein Expr. Purif. 3:263-281 [1992]), protein A domains that allow purification on immobilized immunoglobulin, and the domain utilized in the FLAGS extension/affinity purification system. The inclusion of a cleavable linker sequence such as Factor XA or enterokinase (e.g., sequences available from Invitrogen, San Diego, CA) between the purification domain and the heterologous protein also find use to facilitate purification.

A variety of methods can be used to determine the level of production of a mature protease (e.g., one or more mature subtilisin variant described herein) in a host cell. Such methods include, but are not limited to, for example, methods that utilize either polyclonal or monoclonal antibodies specific for the protease. Exemplary methods include, but are not limited to enzyme-linked immunosorbent assays (ELISA), radioimmunoassays (RIA), fluorescent immunoassays (FIA), and fluorescent activated cell sorting (FACS). These and other assays are well known in the art (*See e.g.,* Maddox et al., J. Exp. Med. 158:1211 (1983)).

Also described herein are methods for making or producing a one or more mature subtilisin variant described herein. A mature subtilisin variant does not include a signal peptide or a propeptide sequence. Some methods comprise making or producing one or more subtilisin variant described herein in a recombinant bacterial host cell, such as for example, a *Bacillus sp.* cell (e.g., a *B. subtilis* cell). Also described herein is a method of producing one or more subtilisin variant described herein, the method comprising cultivating a recombinant host cell comprising a recombinant expression vector comprising a nucleic acid encoding one or more subtilisin variant described herein under conditions conducive to the production of the variant. Some such methods further comprise recovering the variant from the culture.

Also described herein are methods of producing one or more subtilisin variant described herein, the methods comprising: (a) introducing a recombinant expression vector comprising a nucleic acid encoding the variant into a population of cells (e.g., bacterial cells, such as *B. subtilis* cells); and (b) culturing the cells in a culture medium under conditions conducive to produce the variant encoded by the expression vector. Some such methods further comprise: (c) isolating the variant from the cells or from the culture medium.

Unless otherwise noted, all component or composition levels provided herein are made in reference to the active level of that component or composition, and are exclusive of impurities, for example, residual solvents or by-products, which may be present in commercially available sources. Enzyme components weights are based on total active protein. All percentages and ratios are calculated by weight unless otherwise indicated. All percentages and ratios are calculated based on the total composition unless otherwise indicated. Compositions described herein include cleaning compositions, such as detergent compositions. In the exemplified detergent compositions, the enzyme levels are expressed by pure enzyme by weight of the total composition and unless otherwise specified, the detergent ingredients are expressed by weight of the total compositions.

In one embodiment, the one or more subtilisin variant as set out in the claims is useful in cleaning applications, such as, e.g., but not limited to, cleaning dishware or tableware items, fabrics, and items having hard surfaces (e.g., the hard surface of a table, table top, wall, furniture item, floor, and ceiling). Another embodiment is directed to a composition comprising one or more subtilisin variant as set out in the claims. In some embodiments, the composition is a cleaning composition. In other embodiments, the composition is a detergent composition. In yet other embodiments, the composition is selected from a laundry detergent composition, an automatic dishwashing (ADW) composition, a hand (manual) dishwashing detergent composition, a hard surface cleaning composition, an eyeglass cleaning composition, and a personal care cleaning composition. In still other embodiments, the composition is a laundry detergent composition, and ADW composition, or a hand (manual) dishwashing detergent composition. Even still further embodiments are directed to fabric cleaning compositions, while other embodiments are directed to non-fabric cleaning compositions. In some embodiments, the cleaning composition is borate-free. In some embodiments, the cleaning composition is phosphate-free. In some embodiments, the composition comprises one or more subtilisin variant described herein and one or more of an excipient; adjunct material; and/or additional enzyme.

In another embodiment, one or more composition described herein is in a form selected from gel, tablet, powder, granular, solid, liquid, unit dose, and combinations thereof. In some embodiments, the cleaning composition describe herein is in a unit dose form. In other embodiments, the unit does form is selected from pills, tablets, capsules, gelcaps, sachets, pouches, multi-compartment pouches, and pre-measured powders or liquids. In some embodiments, the unit dose format is designed to provide controlled release of the ingredients within a multi-compartment pouch (or other unit dose format). Suitable unit dose and controlled release formats are described, for example, in EP 2100949; WO 02102955; US4765916; US4972017; and WO04/111178. In some embodiments, the unit dose form is a tablet or powder contained in a water-soluble film or pouch.

Exemplary laundry detergent compositions include, but are not limited to, for example, liquid and powder laundry detergent compositions. Exemplary hard surface cleaning compositions include, but not limited to, for example, compositions used to clean the hard surface of a non-dishware item, non-tableware item, table, table top, furniture item, wall, floor, and ceiling. Exemplary hard surface cleaning compositions are described, for example, in USPNs 6610642, 6376450, and 6376450. Exemplary personal care compositions include, but are not limited to, compositions used to clean dentures, teeth, hair, contact lenses, and skin. Exemplary components of such oral care composition include those described in, for example, US6376450.

In some embodiments, one or more subtilisin variant as set out in the claims cleans at low temperatures. In other embodiments, one or more composition as set out in the claims cleans at low temperatures. In other embodiments, one or more composition as set out in the claims comprises an effective amount of one or more subtilisin variant described herein as useful or effective for cleaning a surface in need of proteinaceous stain removal

In some embodiments, adjunct materials are incorporated, for example, to assist or enhance cleaning performance; for treatment of the substrate to be cleaned; or to modify the aesthetics of the cleaning composition as is the case with perfumes, colorants, dyes or the like. One embodiment is directed to a composition comprising one or more adjunct material and one or more subtilisin variant as set out in the claims. Another embodiment is directed to a composition comprising one or more adjunct material and one or more subtilisin variant as set out in the claims, wherein the adjunct material is selected from a bleach catalyst, an additional enzyme, an enzyme stabilizing system, a chelant, an optical brightener, a soil release polymer, a dye transfer agent, a dispersants, a suds suppressor, a dye, a perfume, a colorant, a filler, a photoactivator, a fluorescer, a fabric conditioner, a hydrolysable surfactant, a preservative, an anti-oxidant, an anti-shrinkage agent, an anti-wrinkle agent, a germicide, a fungicide, a color speckle, a silver care agent, an anti-tarnish agent, an anticorrosion agent, an alkalinity source, a solubilizing agent, a carrier, a processing aid, a pigment, a pH control agent, a surfactant, a builder, a chelating agent, a dye transfer inhibiting agent, a deposition aid, a dispersant, a catalytic material, a bleach activator, a bleach booster, a hydrogen peroxide, a source of hydrogen peroxide, a preformed peracid, a polymeric dispersing agent, a clay soil removal/anti-redeposition agent, a structure elasticizing agent, a fabric softener, a carrier, a hydrotrope, a processing aid, a pigment, and combinations thereof. Exemplary adjunct materials and levels of use are found in USPNs 5576282; 6306812; 6326348; 6610642; 6605458; 5705464; 5710115; 5698504; 5695679; 5686014 and 5646101. In embodiments in which one or more cleaning adjunct material is not compatible with one or more subtilisin variant as set out in the claims, methods are employed to keep the adjunct material and variant(s) separated (i.e., not in contact with each other) until combination of the two components is appropriate. Such separation methods include any suitable method known in the art (e.g., gelcaps, encapsulation, tablets, physical separation, etc.).

Some embodiments are directed to cleaning additive products comprising one or more subtilisin variant as set out in the claims. In some embodiments, the additive is packaged in dosage form for addition to a cleaning process. In some embodiments, the additive is packaged in a dosage form for addition to a cleaning process where a source of peroxygen is employed and increased bleaching effectiveness is desired.

Exemplary fillers or carriers for granular compositions include, but are not limited to, for example, various salts of sulfate, carbonate and silicate; talc; and clay. Exemplary fillers or carriers for liquid compositions include, but are not limited to, for example, water or low molecular weight primary and secondary alcohols including polyols and diols (e.g., methanol, ethanol, propanol and isopropanol). In some embodiments, the compositions contain from about 5% to about 90% of such filler or carrier. Acidic fillers may be included in such compositions to reduce the pH of the resulting solution in the cleaning method or application.

In one embodiment, one or more cleaning composition as set out in the claims comprises an effective amount of one or more subtilisin variant described herein, alone or in combination with one or more additional enzyme. Typically, a cleaning composition comprises at least about 0.0001 to about 20 wt %, from about 0.0001 to about 10 wt %, from about 0.0001 to about 1 wt %, from about 0.001 to about 1 wt %, or from about 0.01 to about 0.1 wt % of one or more protease. In another embodiment, one or more cleaning composition described herein comprises from about 0.01 to about 10 mg, about 0.01 to about 5 mg, about 0.01 to about 2 mg, about 0.01 to about 1 mg, about 0.5 to about 10 mg, about 0.5 to about 5 mg, about 0.5 to about 4 mg, about 0.5 to about 4 mg, about 0.5 to about 3 mg, about 0.5 to about 2 mg, about 0.5 to about 1 mg, about 0.1 to about 10 mg, about 0.1 to about 5 mg, about 0.1 to about 4 mg, about 0.1 to about 3 mg, about 0.1 to about 2 mg, about 0.1 to about 2 mg, about 0.1 to about 1 mg, or about 0.1 to about 0.5 mg of one or more protease per gram of composition.

The cleaning compositions herein are typically formulated such that, during use in aqueous cleaning operations, the wash water will have a pH of from about 4.0 to about 11.5, or even from about 5.0 to about 11.5, or even from about 5.0 to about 8.0, or even from about 7.5 to about 10.5. Liquid product formulations are typically formulated to have a pH from about 3.0 to about 9.0 or even from about 3 to about 5. Granular laundry products are typically formulated to have a pH from about 9 to about 11. In some embodiments, the cleaning compositions of the present invention can be formulated to have an alkaline pH under wash conditions, such as a pH of from about 8.0 to about 12.0, or from about 8.5 to about 11.0, or from about 9.0 to about 11.0. In some embodiments, the cleaning compositions of the present invention can be formulated to have a neutral pH under wash conditions, such as a pH of from about 5.0 to about 8.0, or from about 5.5 to about 8.0, or from about 6.0 to about 8.0, or from about 6.0 to about 7.5. In some embodiments, the neutral pH conditions can be measured when the cleaning composition is dissolved 1:100 (wt:wt) in de-ionized water at 20°C, measured using a conventional pH meter. Techniques for controlling pH at recommended usage levels include the use of buffers, alkalis, acids, etc., and are well known to those skilled in the art.

In some embodiments, one or more subtilisin variant of the present invention is encapsulated to protect it during storage from the other components in the composition and/or control the availability of the variant during cleaning. In some embodiments, encapsulation enhances the performance of the variant and/or additional enzyme. In some embodiments, the encapsulating material typically encapsulates at least part of the subtilisin variant of the present invention. Typically, the encapsulating material is water-soluble and/or water-dispersible. In some embodiments, the encapsulating material has a glass transition temperature (Tg) of 0°C or higher. Exemplary encapsulating material includes, but is not limited to, carbohydrates, natural or synthetic gums, chitin, chitosan, cellulose and cellulose derivatives, silicates, phosphates, borates, polyvinyl alcohol, polyethylene glycol, paraffin waxes, and combinations thereof. When the encapsulating material is a carbohydrate, it is typically selected from monosaccharides, oligosaccharides, polysaccharides, and combinations thereof. In some embodiments, the encapsulating material is a starch (*See e.g.,* EP0922499, US 4,977,252, US 5,354,559, and US 5,935,826). In some embodiments, the encapsulating material is a microsphere made from plastic such as thermoplastics, acrylonitrile, methacrylonitrile, polyacrylonitrile, polymethacrylonitrile and mixtures thereof. Exemplary commercial microspheres include, but are not limited to EXPANCEL^{®} (Akzo Nobel Chemicals International, B.V.); PM6545, PM6550, PM7220, PM7228, and EXTENDOSPHERES^{®} (Sphere One Inc.); and LUXSIL^{®}, Q-CEL^{®}, and SPHERICEL^{®} (Potters Industries LLC).

There are a variety of wash conditions including varying detergent formulations, wash water volumes, wash water temperatures, and lengths of wash time to which one or more subtilisin variant described herein may be exposed. A low detergent concentration system is directed to wash water containing less than about 800 ppm detergent components. A medium detergent concentration system is directed to wash containing between about 800 ppm and about 2000ppm detergent components. A high detergent concentration system is directed to wash water containing greater than about 2000 ppm detergent components. The "cold water washing" described herein utilizes "cold water detergent" suitable for washing at temperatures from about 10°C to about 40°C, from about 20°C to about 30°C, or from about 15°C to about 25°C, as well as all other combinations within the range of about 15°C to about 35°C or 10°C to 40°C.

Different geographies have different water hardness. Hardness is a measure of the amount of calcium (Ca²⁺) and magnesium (Mg²⁺) in the water. Water hardness is usually described in terms of the grains per gallon (gpg) mixed Ca²⁺/Mg²⁺. Most water in the United States is hard, but the degree of hardness varies. Moderately hard (60-120 ppm) to hard (121-181 ppm) water has 60 to 181 ppm (ppm can be converted to grains per U.S. gallon by dividing ppm by 17.1) of hardness minerals.

| Water | Grains per gallon | Parts per million |
|---|---|---|
| Soft | less than 1.0 | less than 17 |
| Slightly hard | 1.0 to 3.5 | 17 to 60 |
| Moderately hard | 3.5 to 7.0 | 60 to 120 |
| Hard | 7.0 to 10.5 | 120 to 180 |
| Very hard | greater than 10.5 | greater than 180 |

Other embodiments are directed to one or more cleaning composition comprising from about 0.00001 % to about 10% by weight composition of one or more subtilisin variant of the present invention and from about 99.999% to about 90.0% by weight composition of one or more adjunct material. In another embodiment, the cleaning composition comprises from about 0.0001 % to about 10%, about 0.001% to about 5%, about 0.001% to about 2%, or about 0.005% to about 0.5% by weight composition of one or more subtilisin variant of the present invention and from about 99.9999% to about 90.0%, about 99.999 % to about 98%, about 99.995% to about 99.5% by weight composition of one or more adjunct material.

In other embodiments, the composition described herein comprises one or more subtilisin variant of the present invention and one or more additional enzyme. The one or more additional enzyme is selected from acyl transferases, alpha-amylases, beta-amylases, alpha-galactosidases, arabinosidases, aryl esterases, beta-galactosidases, carrageenases, catalases, cellobiohydrolases, cellulases, chondroitinases, cutinases, endo-beta-1, 4-glucanases, endo-beta-mannanases, esterases, exo-beta-mannanases, galactanases, glucoamylases, hemicellulases, hyaluronidases, keratinases, laccases, lactases, ligninases, lipases, lipoxygenases, mannanases, metalloproteases, oxidases, pectate lyases, pectin acetyl esterases, pectinases, pentosanases, perhydrolases, peroxidases, phenoloxidases, phosphatases, phospholipases, phytases, polygalacturonases, additional proteases, pullulanases, reductases, rhamnogalacturonases, beta-glucanases, tannases, transglutaminases, xylan acetyl-esterases, xylanases, xyloglucanases, and xylosidases, or any combinations or mixtures thereof. Some embodiments are directed to a combination of enzymes (i.e., a "cocktail") comprising conventional enzymes like amylase, lipase, cutinase and/or cellulase in conjunction with one or more subtilisin variant described herein and/or one or more additional protease.

In another embodiment, one or more composition as set out in the claims comprises one or more subtilisin variant described herein and one or more additional protease. In one embodiment, the additional protease is a serine protease. In another embodiment, the additional protease is an alkaline microbial protease or a trypsin-like protease. Suitable additional proteases include those of animal, vegetable or microbial origin. In some embodiments, the additional protease is a microbial protease. In other embodiments, the additional protease is a chemically or genetically modified mutants. In another embodiment, the additional protease is an alkaline microbial protease or a trypsin-like protease. Exemplary alkaline proteases include subtilisins derived from, for example, *Bacillus* (e.g., subtilisin, *lentus, amyloliquefaciens,* subtilisin Carlsberg, subtilisin 309, subtilisin 147 and subtilisin 168). Exemplary additional proteases include but are not limited to those described in WO95/23221, WO 92/21760, WO2008010925, WO20100566356, WO2011072099, WO201113022, WO2011140364, WO 2012151534, WO2015038792, WO2015089441, WO2015089447, WO2015143360, WO2016001449, WO2016001450, WO2016061438, WO2016069544,WO2016069548, WO2016069552, WO 2016069557, WO2016069563, WO2016069569, WO2016087617, WO2016087619, WO2016145428, WO2016174234, WO2016183509, WO2016202835, WO2016205755, US 2008/0090747, US 5,801,039, US 5,340,735, US 5,500,364, US 5,855,625, RE 34,606, US 5,955,340, US 5,700,676, US 6,312,936, US 6,482,628, US8530219, US Provisional Appl Nos. 62/331282, 62/343618, 62/351649, 62/437171, 62/437174, and 62/437509, and PCT Appl Nos. PCT/CN2017/076749 and, as well as metalloproteases described in WO 2007/044993, WO 2009/058303, WO 2009/058661, WO 2014/071410, WO 2014/194032, WO 2014/194034, WO 2014/194054, and WO 2014/194117. Exemplary additional proteases include, but are not limited to trypsin (e.g., of porcine or bovine origin) and the *Fusarium* protease described in WO 89/06270. Exemplary commercial proteases include, but are not limited to MAXATASE, MAXACAL, MAXAPEM, OPTICLEAN^{®}, OPTIMASE^{®}, PROPERASE^{®}, PURAFECT^{®}, PURAFECT^{®} OXP, PURAMAX^{®}, EXCELLASE^{®}, PREFERENZ^{™} proteases (e.g. P100, P110, P280), EFFECTENZ^{™} proteases (e.g. P1000, P1050, P2000), EXCELLENZ^{™} proteases (e.g. P1000), ULTIMASE^{®}, and PURAFAST (Danisco US); ALCALASE^{®}, ALCALASE^{®} ULTRA, BLAZE^{®}, BLAZE^{®} EVITY^{®}, BLAZE^{®} EVITY^{®} 16L, CORONASE^{®}, SAVINASE^{®}, SAVINASE^{®} ULTRA, SAVINASE^{®} EVITY^{®}, SAVINASE^{®} EVERIS^{®}, PRIMASE, DURAZYM, POLARZYME^{®}, OVOZYME^{®}, KANNASE^{®}, LIQUANASE^{®}, EVERIS^{®}, NEUTRASE^{®}, PROGRESS UNO^{®}, RELASE^{®} and ESPERASE^{®} (Novozymes); BLAP^{™} and BLAP^{™} variants (Henkel); LAVERGY^{™} PRO 104 L (BASF), and KAP^{®} (*B. alkalophilus* subtilisin) (Kao).

Another embodiment is directed to a composition comprising one or more subtilisin variant as set out in the claims and one or more lipase. In some embodiments, the composition comprises from about 0.00001 % to about 10%, about 0.0001 % to about 10%, about 0.001% to about 5%, about 0.001% to about 2%, or about 0.005% to about 0.5% lipase by weight composition. An exemplary lipase can be a chemically or genetically modified mutant. Exemplary lipases include, but are not limited to, e.g., those of bacterial or fungal origin, such as, e.g., *H. lanuginosa* lipase (*see, e.g.,* EP 258068 and EP 305216), *T. lanuginosus* lipase (*see, e.g.,* WO2014/059360 and WO2015/010009), *Rhizomucor miehei* lipase (*see, e.g.,* EP 238023), *Candida* lipase, such as *C*. *antarctica* lipase (e.g., C. *antarctica* lipase A or B) (*see, e.g.,* EP 214761), *Pseudomonas* lipases such as *P. alcaligenes* and *P. pseudoalcaligenes* lipase (*see, e.g.,* EP 218272), *P. cepacia* lipase (*see, e.g.,* EP 331376), *P. stutzeri* lipase (*see, e.g.,* GB 1,372,034), *P. fluorescens* lipase, *Bacillus* lipase (e.g., *B. subtilis* lipase (Dartois et al., Biochem. Biophys. Acta 1131:253-260 (1993)), *B. stearothermophilus* lipase (*see, e.g.,* JP 64/744992), and *B. pumilus* lipase *(see, e.g.,* WO 91/16422)). Exemplary cloned lipases include, but not limited to *Penicillium camembertii* lipase (*See,* Yamaguchi et al., Gene 103:61-67 (1991)), *Geotricum candidum* lipase (*See,* Schimada et al., J. Biochem., 106:383-388 (1989)), and various *Rhizopus* lipases, such as, *R. delemar* lipase (*See,* Hass et al., Gene 109:117-113 (1991)), *R*. *niveus* lipase (Kugimiya et al., Biosci. Biotech. Biochem. 56:716-719 (1992)) and *R. oryzae* lipase. Other lipolytic enzymes, such as cutinases, may also find use in one or more composition of the present invention, including, but not limited to, e.g., cutinase derived from *Pseudomonas mendocina (see,* WO 88/09367) and/or *Fusarium solani pisi* (*see,* WO 90/09446). Exemplary commercial lipases include, but are not limited to M1 LIPASE, LUMA FAST, and LIPOMAX (Genencor); LIPEX^{®}, LIPOCLEAN^{®}, LIPOLASE^{®} and LIPOLASE^{®} ULTRA (Novozymes); and LIPASE P (Amano Pharmaceutical Co. Ltd).

A still further embodiment is directed to a composition comprising one or more subtilisin variant as set out in the claims and one or more amylase. In one embodiment, the composition comprises from about 0.00001 % to about 10%, about 0.0001 % to about 10%, about 0.001% to about 5%, about 0.001% to about 2%, or about 0.005% to about 0.5% amylase by weight composition. Any amylase (e.g., alpha and/or beta) suitable for use in alkaline solutions may be useful to include in such composition. An exemplary amylase can be a chemically or genetically modified mutant. Exemplary amylases include, but are not limited to those of bacterial or fungal origin, such as, for example, amylases described in GB 1,296,839, WO91/00353, WO94/02597, WO94/183314, WO95/10603, WO95/26397, WO95/35382, WO 96/05295, WO96/23873, WO96/23874, WO96/30481, WO97/10342, WO97/41213, WO97/43424, WO 98/13481, WO98/26078, WO99/02702, WO99/09183, WO99/19467, WO99/23211, WO99/29876, WO 99/42567, WO99/43793, WO99/43794, WO99/46399, WO00/29560, WO00/60058, WO00/60059, WO 00/60060, WO01/14532, WO01/34784, WO01/64852, WO01/66712, WO01/88107, WO01/96537, WO 02/092797, WO02/10355, WO02/31124, WO2004055178, WO2004113551, WO2005001064, WO 2005003311, WO2005018336, WO2005019443, WO2005066338, WO2006002643, WO2006012899, WO2006012902, WO2006031554, WO2006063594, WO2006066594, WO2006066596, WO 2006136161, WO2008000825, WO2008088493, WO2008092919, WO2008101894, WO2008112459, WO2009061380, WO2009061381, WO2009100102, WO2009140504, WO2009149419, WO 2010059413, WO2010088447, WO2010091221, WO2010104675, WO2010115021, WO2010115028, WO2010117511, WO2011076123, WO2011076897, WO2011080352, WO2011080353, WO 2011080354, WO2011082425, WO2011082429, WO2011087836, WO2011098531, WO2013063460, WO2013184577, WO2014099523, WO2014164777, and WO2015077126. Exemplary commercial amylases include, but are not limited to AMPLIFY^{®}, AMPLIFY PRIME^{®}, BAN DURAMYL^{®}, TERMAMYL^{®}, TERMAMYL^{®} ULTRA, FUNGAMYL^{®}, STAINZYME^{®}, STAINZYME^{®} PLUS, STAINZYME^{®} ULTRA, and STAINZYME^{®} EVITY^{®} (Novozymes); EFFECTENZ^{™} S 1000, POWERASE^{®}, PREFERENZ^{™} S 100, PREFERENZ^{™} S 110, EXCELLENZ^{™} S 2000, RAPIDASE^{®} and MAXAMYL^{®} P (Danisco US).

Yet a still further embodiment is directed to a composition comprising one or more subtilisin variant as set out in the claims and one or more cellulase. In one embodiment, the composition comprises from about 0.00001 % to about 10%, 0.0001 % to about 10%, about 0.001% to about 5%, about 0.001% to about 2%, or about 0.005% to about 0.5% cellulase by weight of composition. Any suitable cellulase may find used in a composition of the present invention. An exemplary cellulase can be a chemically or genetically modified mutant. Exemplary cellulases include but are not limited, to those of bacterial or fungal origin, such as, for example, is described in WO2005054475, WO2005056787, US 7,449,318, US 7,833,773, US 4,435,307; EP 0495257; and US Provisional Appl. Nos. 62/296,678 and 62/435340. Exemplary commercial cellulases include, but are not limited to, CELLUCLEAN^{®}, CELLUZYME^{®}, CAREZYME^{®}, CAREZYME^{®} PREMIUM, ENDOLASE^{®}, and RENOZYME^{®} (Novozymes); REVITALENZ^{®}100, REVITALENZ^{®} 200/220 and REVITALENZ^{®} 2000 (Danisco US); and KAC-500(B) (Kao Corporation). In some embodiments, cellulases are incorporated as portions or fragments of mature wild-type or variant cellulases, wherein a portion of the N-terminus is deleted (*see, e.g.,* US 5,874,276).

An even still further embodiment is directed to a composition comprising one or more subtilisin variant as set out in the claims and one or more mannanase. In one embodiment, the composition comprises from about 0.00001 % to about 10%, about 0.0001 % to about 10%, about 0.001% to about 5%, about 0.001% to about 2%, or about 0.005% to about 0.5% mannanase by weight composition. An exemplary mannanase can be a chemically or genetically modified mutant. Exemplary mannanases include, but are not limited to, those of bacterial or fungal origin, such as, for example, as is described in WO2016007929; USPNs 6566114, 6602842, and 6440991; and WO2017/079756 and WO2017/079751. Exemplary commercial mannanases include, but are not limited to MANNAWAY^{®} (Novozymes); and EFFECTENZ^{™} M 1000, PREFERENZ^{®} M 100, MANNASTAR^{®}, and PURABRITE (Danisco US).

A yet even still further embodiment is directed to a composition comprising one or more subtilisin variant as set out in the claims and one or more peroxidase and/or oxidase enzyme. In one embodiment, the composition comprises from about 0.00001 % to about 10%, about 0.0001 % to about 10%, about 0.001% to about 5%, about 0.001% to about 2%, or about 0.005% to about 0.5% peroxidase or oxidase by weight composition. A peroxidase may be used in combination with hydrogen peroxide or a source thereof (e.g., a percarbonate, perborate or persulfate) and an oxidase may be used in combination with oxygen. Peroxidases and oxidases are used for "solution bleaching" (i.e., to prevent transfer of a textile dye from a dyed fabric to another fabric when the fabrics are washed together in a wash liquor), alone or in combination with an enhancing agent (*see, e.g.,* WO94/12621 and WO95/01426). An exemplary peroxidase and/or oxidase can be a chemically or genetically modified mutant. Exemplary peroxidases/oxidases include, but are not limited to those of plant, bacterial, or fungal origin.

Another embodiment is directed to a composition comprising one or more subtilisin variant as set out in the claims and one or more one or more perhydrolase, such as, for example, is described in WO 2005056782, WO2007106293, WO2008063400, WO2008106214, and WO2008106215.

In yet another embodiment, the subtilisin variant as set out in the claims and one or more additional enzyme contained in one or more composition described herein may each independently range to about 10%, wherein the balance of the cleaning composition is one or more adjunct material.

In some embodiments, one or more composition as set out in the claims finds use as a detergent additive, wherein said additive is in a solid or liquid form. Such additive products are intended to supplement and/or boost the performance of conventional detergent compositions and can be added at any stage of the cleaning process. In some embodiments, the density of the laundry detergent composition ranges from about 400 to about 1200 g/liter, while in other embodiments it ranges from about 500 to about 950 g/liter of composition measured at 20°C.

Some embodiments are directed to a laundry detergent composition comprising one or more subtilisin variant of the present invention and one or more adjunct materials selected from surfactants, builder compounds, polymeric compounds, bleaching agents, additional enzymes, suds suppressors, dispersants, lime-soap dispersants, soil suspension agents, anti-redeposition agents, corrosion inhibitors, and combinations thereof. In some embodiments, the laundry compositions also contain softening agents.

Further embodiments are directed to manual dishwashing composition comprising one or more subtilisin variant of the present invention and one or more adjunct materials selected from surfactants, organic polymeric compounds, suds enhancing agents, group II metal ions, solvents, hydrotropes, and additional enzymes.

Other embodiments are directed to one or more composition as set out in the claims, wherein said composition is a compact granular fabric cleaning composition that finds use in laundering colored fabrics or provides softening through the wash capacity, or is a heavy duty liquid (HDL) fabric cleaning composition. Exemplary fabric cleaning compositions and/or processes for making are described in USPNs 6,610,642 and 6,376,450. Other exemplary cleaning compositions are described, for example, in USPNs 6,605,458; 6,294,514; 5,929,022; 5,879,584; 5,691,297; 5,565,145; 5,574,005; 5,569,645; 5,565,422; 5,516,448; 5,489,392; and 5,486,303; 4,968,451; 4,597,898; 4,561,998; 4,550,862; 4,537,706; 4,515,707; and 4,515,705.

In some embodiments, the cleaning compositions of the present invention comprise an acidifying particle or an amino carboxylic builder. Examples of an amino carboxylic builder include aminocarboxylic acids, salts and derivatives thereof. In some embodiment, the amino carboxylic builder is an aminopolycarboxylic builder, such as glycine-N,N-diacetic acid or derivative of general formula MOOC-CHR-N(CH₂COOM)₂ where R is C₁₋₁₂alkyl and M is alkali metal. In some embodiments, the amino carboxylic builder can be methylglycine diacetic acid (MGDA), GLDA (glutamic-N,N-diacetic acid), iminodisuccinic acid (IDS), carboxymethyl inulin and salts and derivatives thereof, aspartic acid-N-monoacetic acid (ASMA), aspartic acid-N,N-diacetic acid (ASDA), aspartic acid-N-monopropionic acid (ASMP), iminodisuccinic acid (IDA), N-(2-sulfomethyl) aspartic acid (SMAS), N-(2-sulfoethyl)aspartic acid (SEAS), N-(2-sulfomethyl)glutamic acid (SMGL), N-(2-sulfoethyl) glutamic acid (SEGL), IDS (iminodiacetic acid) and salts and derivatives thereof such as N-methyliminodiacetic acid (MIDA) , alpha-alanine-N,N-diacetic acid (alpha-ALDA) , serine-N,N-diacetic acid (SEDA), isoserine-N,Ndiacetic acid (ISDA) , phenylalanine-N,N-diacetic acid (PHDA), anthranilic acid-N,N-diacetic acid (ANDA), sulfanilic acid-N,N-diacetic acid (SLDA), taurine-N,N-diacetic acid (TUDA) and sulfomethyl-N,N-diacetic acid (SMDA) and alkali metal salts and derivative thereof. In some embodiments, the acidifying particle has a weight geometric mean particle size of from about 400µ to about 1200µ and a bulk density of at least 550 g/L. In some embodiments, the acidifying particle comprises at least about 5% of the builder.

In some embodiments, the acidifying particle can comprise any acid, including organic acids and mineral acids. Organic acids can have one or two carboxyls and in some instances up to 15 carbons, especially up to 10 carbons, such as formic, acetic, propionic, capric, oxalic, succinic, adipic, maleic, fumaric, sebacic, malic, lactic, glycolic, tartaric and glyoxylic acids. In some embodiments, the acid is citric acid. Mineral acids include hydrochloric and sulphuric acid. In some instances, the acidifying particle is a highly active particle comprising a high level of amino carboxylic builder. Sulphuric acid has also been found to further contribute to the stability of the final particle.

Additional embodiments are directed to a cleaning composition comprising one or more subtilisin variant of the present invention and one or more surfactant and/or surfactant system, wherein the surfactant is selected from nonionic surfactants, anionic surfactants, cationic surfactants, ampholytic surfactants, zwitterionic surfactants, semi-polar nonionic surfactants, and mixtures thereof. In some embodiments, the surfactant is present at a level of from about 0.1 to about 60%, while in alternative embodiments the level is from about 1 to about 50%, while in still further embodiments the level is from about 5 to about 40%, by weight of the cleaning composition.

In some embodiments, one or more composition as set out in the claims comprises one or more detergent builders or builder systems. In one embodiment, the composition comprises from about 1%, from about 0.1% to about 80%, from about 3% to about 60%, from about 5% to about 40%, or from about 10% to about 50% builder by weight composition. Exemplary builders include, but are not limited to alkali metal; ammonium and alkanolammonium salts of polyphosphates; alkali metal silicates; alkaline earth and alkali metal carbonates; aluminosilicates; polycarboxylate compounds; ether hydroxypolycarboxylates; copolymers of maleic anhydride with ethylene or vinyl methyl ether, 1, 3, 5-trihydroxy benzene-2, 4, 6-trisulphonic acid, and carboxymethyloxysuccinic acid; ammonium and substituted ammonium salts of polyacetic acids such as ethylenediamine tetraacetic acid and nitrilotriacetic acid; polycarboxylates such as mellitic acid, succinic acid, citric acid, oxydisuccinic acid, polymaleic acid, benzene 1,3,5-tricarboxylic acid, carboxymethyloxysuccinic acid; and soluble salts thereof. In some such compositions, the builders form water-soluble hardness ion complexes (e.g., sequestering builders), such as citrates and polyphosphates, e.g., sodium tripolyphosphate, sodium tripolyphospate hexahydrate, potassium tripolyphosphate, and mixed sodium and potassium tripolyphosphate. Exemplary builders are described in, e.g., EP 2100949. In some embodiments, the builders include phosphate builders and non-phosphate builders. In some embodiments, the builder is a phosphate builder. In some embodiments, the builder is a non-phosphate builder. In some embodiments, the builder comprises a mixture of phosphate and non-phosphate builders. Exemplary phosphate builders include, but are not limited to mono-phosphates, di-phosphates, tri-polyphosphates or oligomeric-poylphosphates, including the alkali metal salts of these compounds, including the sodium salts. In some embodiments, a builder can be sodium tripolyphosphate (STPP). Additionally, the composition can comprise carbonate and/or citrate. Other suitable non-phosphate builders include homopolymers and copolymers of polycarboxylic acids and their partially or completely neutralized salts, monomeric polycarboxylic acids and hydroxycarboxylic acids and their salts. In some embodiments, salts of the above mentioned compounds include the ammonium and/or alkali metal salts, i.e. the lithium, sodium, and potassium salts, including sodium salts. Suitable polycarboxylic acids include acyclic, alicyclic, hetero-cyclic and aromatic carboxylic acids, wherein in some embodiments, they can contain at least two carboxyl groups which are in each case separated from one another by, in some instances, no more than two carbon atoms.

In some embodiments, one or more composition of the present invention comprises one or more chelating agent. In one embodiment, the composition comprises from about 0.1% to about 15% or about 3% to about 10% chelating agent by weight composition. Exemplary chelating agents include, but are not limited to, e.g., copper, iron, manganese, and mixtures thereof.

In some embodiments, one or more composition of the present invention comprises one or more deposition aid. Exemplary deposition aids include, but are not limited to, e.g., polyethylene glycol; polypropylene glycol; polycarboxylate; soil release polymers, such as, e.g., polytelephthalic acid; clays such as, e.g., kaolinite, montmorillonite, atapulgite, illite, bentonite, and halloysite; and mixtures thereof.

In other embodiments, one or more composition of the present invention comprises one or more anti-redeposition agent or non-ionic surfactant (which can prevent the re-deposition of soils) (*see, e.g.,* EP 2100949). For example, in ADW compositions, non-ionic surfactants find use for surface modification purposes, in particular for sheeting, to avoid filming and spotting and to improve shine. These non-ionic surfactants also find use in preventing the re-deposition of soils. In some embodiments, the non-ionic surfactant can be ethoxylated nonionic surfactants, epoxy-capped poly(oxyalkylated) alcohols and amine oxides surfactants.

In some embodiments, one or more composition of the present invention comprises one or more dye transfer inhibiting agent. Exemplary polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones, polyvinylimidazoles, and mixtures thereof. In one embodiment, the composition comprises from about 0.0001% to about 10%, about 0.01% to about 5%, or about 0.1% to about 3% dye transfer inhibiting agent by weight composition.

In some embodiments, one or more composition of the present invention comprises one or more silicate. Exemplary silicates include, but are not limited to, sodium silicates, e.g., sodium disilicate, sodium metasilicate, and crystalline phyllosilicates. In some embodiments, silicates are present at a level of from about 1% to about 20% or about 5% to about 15% by weight of the composition.

In some still additional embodiments, one or more composition of the present invention comprises one or more dispersant. Exemplary water-soluble organic materials include, but are not limited to, e.g., homo- or co-polymeric acids or their salts, in which the polycarboxylic acid comprises at least two carboxyl radicals separated from each other by not more than two carbon atoms.

In some further embodiments, one or more composition of the present invention comprises one or more enzyme stabilizer. In some embodiments, the enzyme stabilizer is water-soluble sources of calcium and/or magnesium ions. In some embodiments, the enzyme stabilizers include oligosaccharides, polysaccharides, and inorganic divalent metal salts, including alkaline earth metals, such as calcium salts. In some embodiments, the enzymes employed herein are stabilized by the presence of water-soluble sources of zinc (II), calcium (II) and/or magnesium (II) ions in the finished compositions that provide such ions to the enzymes, as well as other metal ions (e.g., barium (II), scandium (II), iron (II), manganese (II), aluminum (III), tin (II), cobalt (II), copper (II), nickel (II), and oxovanadium (IV)). Chlorides and sulfates also find use in some embodiments. Exemplary oligosaccharides and polysaccharides (e.g., dextrins) are described, for example, in WO 07/145964. In some embodiments, reversible protease inhibitors also find use, such as boron-containing compounds (e.g., borate, 4-formyl phenyl boronic acid) and/or tripeptide aldehydes. In some embodiments of the present invention, the composition comprises at least one ion selected from calcium and zinc.

In some embodiments, one or more composition of the present invention comprises one or more bleach, bleach activator, and/or bleach catalyst. In some embodiments, one or more composition of the present invention comprises one or more inorganic and/or organic bleaching compound. Exemplary inorganic bleaches include, but are not limited to perhydrate salts, e.g., perborate, percarbonate, perphosphate, persulfate, and persilicate salts. In some embodiments, inorganic perhydrate salts are alkali metal salts. In some embodiments, inorganic perhydrate salts are included as the crystalline solid, without additional protection, although in some other embodiments, the salt is coated. Bleach activators are typically organic peracid precursors that enhance the bleaching action in the course of cleaning at temperatures of 60°C and below. Exemplary bleach activators include compounds which, under perhydrolysis conditions, give aliphatic peroxoycarboxylic acids having from about 1 to about 10 carbon atoms or about 2 to about 4 carbon atoms, and/or optionally substituted perbenzoic acid. Exemplary bleach activators ae described, for example, in EP 2100949. Exemplary bleach catalysts include, but are not limited to, manganese triazacyclononane and related complexes, as well as cobalt, copper, manganese, and iron complexes. Additional exemplary bleach catalysts are described, for example, in US 4,246,612; US 5,227,084; US 4,810,410; WO 99/06521; and EP 2100949.

In some embodiments, one or more composition of the present invention comprises one or more catalytic metal complexes. In some embodiments, a metal-containing bleach catalyst finds use. In some embodiments, the metal bleach catalyst comprises a catalyst system comprising a transition metal cation of defined bleach catalytic activity (e.g., copper, iron, titanium, ruthenium, tungsten, molybdenum, or manganese cations), an auxiliary metal cation having little or no bleach catalytic activity (e.g., zinc or aluminum cations), and a sequestrate having defined stability constants for the catalytic and auxiliary metal cations, particularly ethylenediaminetetraacetic acid, ethylenediaminetetra (methylenephosphonic acid) and water-soluble salts thereof (*see, e.g.,* US 4,430,243). In some embodiments, one or more composition of the present invention is catalyzed by means of a manganese compound. Such compounds and levels of use are described, for example, in US 5,576,282. In additional embodiments, cobalt bleach catalysts find use and are included in one or more composition described herein. Various cobalt bleach catalysts are described, for example, in USPNs 5,597,936 and 5,595,967.

In some additional embodiments, one or more composition of the present invention includes a transition metal complex of a macropolycyclic rigid ligand (MRL). As a practical matter, and not by way of limitation, in some embodiments, the compositions and cleaning processes of the present invention are adjusted to provide on the order of at least one part per hundred million, from about 0.005 ppm to about 25 ppm, about 0.05 ppm to about 10 ppm, or about 0.1 ppm to about 5 ppm of active MRL in the wash liquor. Exemplary MRLs include, but are not limited to special ultra-rigid ligands that are cross-bridged, such as, e.g., 5,12-diethyl-1,5,8,12-tetraazabicyclo(6.6.2)hexadecane. Exemplary metal MRLs are described, for example, in WO 2000/32601 and US 6,225,464.

In another embodiment, one or more composition of the present invention comprises one or more metal care agent. In some embodiments, the composition comprises from about 0.1% to about 5% metal care agent by weight composition. Exemplary metal care agents include, for example, aluminum, stainless steel, and non-ferrous metals (e.g., silver and copper). Additional exemplary metal care agents are described, for example, in EP 2100949, WO 94/26860, and WO 94/26859. In some compositions, the metal care agent is a zinc salt.

In some embodiments, the cleaning composition is a high density liquid (HDL) composition comprising one or more subtilisin variant of the present invention. The HDL liquid laundry detergent can comprise a detersive surfactant (10-40%) comprising anionic detersive surfactant selected from a group of linear or branched or random chain, substituted or unsubstituted alkyl sulphates, alkyl sulphonates, alkyl alkoxylated sulphate, alkyl phosphates, alkyl phosphonates, alkyl carboxylates, and/or mixtures thereof; and optionally non-ionic surfactant selected from a group of linear or branched or random chain, substituted or unsubstituted alkyl alkoxylated alcohol, for example, a Cs-C₁₈alkyl ethoxylated alcohol and/or C₆-C₁₂alkyl phenol alkoxylates, optionally wherein the weight ratio of anionic detersive surfactant (with a hydrophilic index (HIc) of from 6.0 to 9) to non-ionic detersive surfactant is greater than 1:1.

The composition of the present invention can comprise optionally, a surfactancy boosting polymer consisting of amphiphilic alkoxylated grease cleaning polymers selected from a group of alkoxylated polymers having branched hydrophilic and hydrophobic properties, such as alkoxylated polyalkylenimines in the range of 0.05wt%-10wt% and/or random graft polymers typically comprising a hydrophilic backbone comprising monomers selected from the group consisting of: unsaturated C₁-C₆carboxylic acids, ethers, alcohols, aldehydes, ketones, esters, sugar units, alkoxy units, maleic anhydride, saturated polyalcohols such as glycerol, and mixtures thereof; and hydrophobic side chain(s) selected from the group consisting of: C₄-C₂₅alkyl group, polypropylene, polybutylene, vinyl ester of a saturated C₂-C₆mono-carboxylic acid, C₁-C₆alkyl ester of acrylic or methacrylic acid, and mixtures thereof.

The composition of the present invention can comprise additional polymers such as soil release polymers including, for example, anionically end-capped polyesters, for example SRP1; polymers comprising at least one monomer unit selected from saccharide, dicarboxylic acid, polyol and combinations thereof, in random or block configuration; ethylene terephthalate-based polymers and co-polymers thereof in random or block configuration, for example, Repel-o-tex SF, SF-2 and SRP6, Texcare SRA100, SRA300, SRN100, SRN170, SRN240, SRN300 and SRN325, Marloquest SL; anti-redeposition polymers (0.1 wt% to 10wt%, including, for example, carboxylate polymers, such as polymers comprising at least one monomer selected from acrylic acid, maleic acid (or maleic anhydride), fumaric acid, itaconic acid, aconitic acid, mesaconic acid, citraconic acid, methylenemalonic acid, and any mixture thereof; vinylpyrrolidone homopolymer; and/or polyethylene glycol with a molecular weight in the range of from 500 to 100,000 Da); cellulosic polymer (including, for example, alkyl cellulose; alkyl alkoxyalkyl cellulose; carboxyalkyl cellulose; alkyl carboxyalkyl cellulose, examples of which include carboxymethyl cellulose, methyl cellulose, methyl hydroxyethyl cellulose, methyl carboxymethyl cellulose; and mixtures thereof); and polymeric carboxylate (such as, for example, maleate/acrylate random copolymer or polyacrylate homopolymer).

The composition of the present invention can further comprise saturated or unsaturated fatty acid, preferably saturated or unsaturated C₁₂-C₂₄fatty acid (0 to 10 wt%); deposition aids (including, for example, polysaccharides, cellulosic polymers, polydiallyl dimethyl ammonium halides (DADMAC), and co-polymers of DADMAC with vinyl pyrrolidone, acrylamides, imidazoles, imidazolinium halides, and mixtures thereof, in random or block configuration; cationic guar gum; cationic cellulose such as cationic hydoxyethyl cellulose; cationic starch; cationic polyacylamides; and mixtures thereof.

The composition of the present invention can further comprise dye transfer inhibiting agents examples of which include manganese phthalocyanine, peroxidases, polyvinylpyrrolidone polymers, polyamine N-oxide polymers, copolymers of N-vinylpyrrolidone and N-vinylimidazole, polyvinyloxazolidones and polyvinylimidazoles and/or mixtures thereof; chelating agents examples of which include ethylene-diamine-tetraacetic acid (EDTA); diethylene triamine penta methylene phosphonic acid (DTPMP); hydroxy-ethane diphosphonic acid (HEDP); ethylenediamine N,N'-disuccinic acid (EDDS); methyl glycine diacetic acid (MGDA); diethylene triamine penta acetic acid (DTPA); propylene diamine tetracetic acid (PDT A); 2-hydroxypyridine-N-oxide (HPNO); or methyl glycine diacetic acid (MGDA); glutamic acid N,N-diacetic acid (N,N-dicarboxymethyl glutamic acid tetrasodium salt (GLDA); nitrilotriacetic acid (NTA); 4,5-dihydroxy-m-benzenedisulfonic acid; citric acid and any salts thereof; N-hydroxyethylethylenediaminetri-acetic acid (HEDTA), triethylenetetraaminehexaacetic acid (TTHA), N-hydroxyethyliminodiacetic acid (HEIDA), dihydroxyethylglycine (DHEG), ethylenediaminetetrapropionic acid (EDTP), and derivatives thereof.

The composition of the present invention may comprise an enzyme stabilizer (examples of which include polyols such as propylene glycol or glycerol, sugar or sugar alcohol, lactic acid, reversible protease inhibitor, boric acid, or a boric acid derivative, e.g., an aromatic borate ester, or a phenyl boronic acid derivative such as 4-formylphenyl boronic acid).

The composition of the present invention can further comprise silicone or fatty-acid based suds suppressors; hueing dyes, calcium and magnesium cations, visual signaling ingredients, anti-foam (0.001 to about 4.0 wt%), and/or structurant/thickener (0.01 to 5 wt%) selected from the group consisting of diglycerides, triglycerides, ethylene glycol distearate, microcrystalline cellulose, cellulose based materials, microfiber cellulose, biopolymers, xanthan gum, gellan gum, and mixtures thereof.

Suitable detersive surfactants also include cationic detersive surfactants (selected from alkyl pyridinium compounds, alkyl quarternary ammonium compounds, alkyl quarternary phosphonium compounds, alkyl ternary sulphonium compounds, and/or mixtures thereof); zwitterionic and/or amphoteric detersive surfactants (selected from alkanolamine sulpho-betaines); ampholytic surfactants; semi-polar non-ionic surfactants; and mixtures thereof.

In some embodiments, the cleaning composition of the present invention is a high density powder (HDD) composition comprising one or more subtilisin variant described herein. The HDD powder laundry detergent can comprise a detersive surfactant including anionic detersive surfactants (selected from linear or branched or random chain, substituted or unsubstituted alkyl sulphates, alkyl sulphonates, alkyl alkoxylated sulphate, alkyl phosphates, alkyl phosphonates, alkyl carboxylates and/or mixtures thereof), non-ionic detersive surfactant (selected from 1 linear or branched or random chain, substituted or unsubstituted C₈-C₁₈ alkyl ethoxylates, and/or C₆-C₁₂ alkyl phenol alkoxylates), cationic detersive surfactants (selected from alkyl pyridinium compounds, alkyl quaternary ammonium compounds, alkyl quaternary phosphonium compounds, alkyl ternary sulphonium compounds, and mixtures thereof), zwitterionic and/or amphoteric detersive surfactants (selected from alkanolamine sulpho-betaines); ampholytic surfactants; semi-polar non-ionic surfactants and mixtures thereof; builders (phosphate free builders, e.g., zeolite builders examples of which include zeolite A, zeolite X, zeolite P and zeolite MAP in the range of 0 to less than 10 wt%); phosphate builders, e.g., sodium tri-polyphosphate in the range of 0 wt% to less than 10 wt%; citric acid, citrate salts and nitrilotriacetic acid or salt thereof in the range of less than 15 wt%; silicate salt (sodium or potassium silicate or sodium meta-silicate in the range of 0 wt% to less than 10 wt% or layered silicate (SKS-6)); carbonate salt (sodium carbonate and/or sodium bicarbonate in the range of 0 to less than 10 wt%); and bleaching agents (photobleaches, e.g., sulfonated zinc phthalocyanines, sulfonated aluminum phthalocyanines, xanthenes dyes, and mixtures thereof); hydrophobic or hydrophilic bleach activators (e.g., dodecanoyl oxybenzene sulfonate, decanoyl oxybenzene sulfonate, decanoyl oxybenzoic acid or salts thereof, 3,5,5-trimethy hexanoyl oxybenzene sulfonate, tetraacetyl ethylene diamine-TAED, and nonanoyloxybenzene sulfonate-NOBS, nitrile quats, and mixtures thereof); hydrogen peroxide; sources of hydrogen peroxide (inorganic perhydrate salts, e.g., mono or tetra hydrate sodium salt of perborate, percarbonate, persulfate, perphosphate, or persilicate); preformed hydrophilic and/or hydrophobic peracids (selected from percarboxylic acids and salts, percarbonic acids and salts, perimidic acids and salts, peroxymonosulfuric acids and salts, and mixtures thereof); and/or bleach catalyst (e.g., imine bleach boosters, such as iminium cations and polyions; iminium zwitterions; modified amines; modified amine oxides; N-sulphonyl imines; N-phosphonyl imines; N-acyl imines; thiadiazole dioxides; perfluoroimines; cyclic sugar ketones and mixtures thereof), metal-containing bleach catalyst (e.g., copper, iron, titanium, ruthenium, tungsten, molybdenum, or manganese cations along with an auxiliary metal cations such as zinc or aluminum and a sequestrate such as ethylenediaminetetraacetic acid, ethylenediaminetetra(methylenephosphonic acid) and water-soluble salts thereof).

The composition of the present invention can further comprise additional detergent ingredients including perfume microcapsules, starch encapsulated perfume accord, hueing agents, additional polymers including fabric integrity and cationic polymers, dye lock ingredients, fabric-softening agents, brighteners (for example C.I. Fluorescent brighteners), flocculating agents, chelating agents, alkoxylated polyamines, fabric deposition aids, and/or cyclodextrin.

In some embodiments, the cleaning composition of the present invention is an ADW detergent composition comprising one or more subtilisin variant described herein. The ADW detergent composition can comprise two or more non-ionic surfactants selected from ethoxylated non-ionic surfactants, alcohol alkoxylated surfactants, epoxy-capped poly(oxyalkylated) alcohols, and amine oxide surfactants present in amounts from 0 to 10% by weight; builders in the range of 5-60% comprising either phosphate (mono-phosphates, di-phosphates, tri-polyphosphates or oligomeric-poylphosphates), sodium tripolyphosphate-STPP or phosphate-free builders (amino acid based compounds, e.g., MGDA (methyl-glycine-diacetic acid) and salts and derivatives thereof, GLDA (glutamic-N,Ndiacetic acid) and salts and derivatives thereof, IDS (iminodisuccinic acid) and salts and derivatives thereof, carboxy methyl inulin and salts and derivatives thereof and mixtures thereof, nitrilotriacetic acid (NTA), diethylene triamine penta acetic acid (DTPA), and B-alaninediacetic acid (B-ADA) and their salts), homopolymers and copolymers of poly-carboxylic acids and their partially or completely neutralized salts, monomeric polycarboxylic acids and hydroxycarboxylic acids and their salts in the range of 0.5 to 50% by weight; sulfonated/carboxylated polymers (provide dimensional stability to the product) in the range of about 0.1 to about 50% by weight; drying aids in the range of about 0.1 to about 10% by weight (selected from polyesters, especially anionic polyesters optionally together with further monomers with 3 to 6 functionalities which are conducive to polycondensation, specifically acid, alcohol or ester functionalities, polycarbonate-, polyurethane- and/or polyurea-polyorganosiloxane compounds or precursor compounds thereof of the reactive cyclic carbonate and urea type); silicates in the range from about 1 to about 20% by weight (sodium or potassium silicates, e.g., sodium disilicate, sodium meta-silicate and crystalline phyllosilicates); bleach-inorganic (e.g., perhydrate salts such as perborate, percarbonate, perphosphate, persulfate and persilicate salts) and organic (e.g., organic peroxyacids including diacyl and tetraacylperoxides, especially diperoxydodecanedioc acid, diperoxytetradecanedioc acid, and diperoxyhexadecanedioc acid); bleach activator-organic peracid precursors in the range from about 0.1 to about 10% by weight; bleach catalysts (selected from manganese triazacyclononane and related complexes, Co, Cu, Mn and Fe bispyridylamine and related complexes, and pentamine acetate cobalt(III) and related complexes); metal care agents in the range from about 0.1 to 5% by weight (selected from benzatriazoles, metal salts and complexes, and silicates); enzymes in the range from about 0.01 to 5.0mg of active enzyme per gram of ADW detergent composition (acyl transferases, alpha-amylases, beta-amylases, alpha-galactosidases, arabinosidases, aryl esterases, beta-galactosidases, carrageenases, catalases, cellobiohydrolases, cellulases, chondroitinases, cutinases, endo-beta-1, 4-glucanases, endo-beta-mannanases, esterases, exo-mannanases, galactanases, glucoamylases, hemicellulases, hyaluronidases, keratinases, laccases, lactases, ligninases, lipases, lipoxygenases, mannanases, oxidases, pectate lyases, pectin acetyl esterases, pectinases, pentosanases, peroxidases, perhydrolases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, xylosidases, and mixtures thereof); and enzyme stabilizer components (selected from oligosaccharides, polysaccharides and inorganic divalent metal salts).

In some embodiments, the composition of the present invention comprises at least one ion selected from calcium and zinc; at least one stabilizer; from about 0.001% to about 1.0 weight % of said variant; at least one bleaching agent; at least one adjunct ingredient; and/or one or more additional enzymes or enzyme derivatives selected from the group consisting of acyl transferases, alpha-amylases, beta-amylases, alpha-galactosidases, arabinosidases, aryl esterases, beta-galactosidases, carrageenases, catalases, cellobiohydrolases, cellulases, chondroitinases, cutinases, endo-beta-1,4-glucanases, endo-beta-mannanases, esterases, exo-beta-mannanases, galactanases, glucoamylases, hemicellulases, hyaluronidases, keratinases, laccases, lactases, ligninases, lipases, lipoxygenases, mannanases, oxidases, pectate lyases, pectin acetyl esterases, pectinases, pentosanases, perhydrolases, peroxidases, phenoloxidases, phosphatases, phospholipases, phytases, polygalacturonases, additional proteases, pullulanases, reductases, rhamnogalacturonases, beta-glucanases, tannases, transglutaminases, xylan acetyl-esterases, xylanases, xyloglucanases, xylosidases, metalloproteases, and combinations thereof.

More embodiments are directed to compositions and methods of treating fabrics (*e.g.,* to desize a textile) using one or more subtilisin variant of the present invention. Fabric-treating methods are well known in the art (*see, e.g.,* US 6,077,316). For example, the feel and appearance of a fabric can be improved by a method comprising contacting the fabric with a variant of the present invention in a solution. The fabric can be treated with the solution under pressure.

One or more subtilisin variant of the present invention can be applied during or after weaving a textile, during the desizing stage, or one or more additional fabric processing steps. During the weaving of textiles, the threads are exposed to considerable mechanical strain. Prior to weaving on mechanical looms, warp yarns are often coated with sizing starch or starch derivatives to increase their tensile strength and to prevent breaking. One or more subtilisin variant of the present invention can be applied during or after weaving to remove the sizing starch or starch derivatives. After weaving, the variant can be used to remove the size coating before further processing the fabric to ensure a homogeneous and wash-proof result. One or more subtilisin variant of the present invention can be used alone or with other desizing chemical reagents and/or desizing enzymes to desize fabrics, including cotton-containing fabrics, as detergent additives, *e.g*., in aqueous compositions. An amylase also can be used in compositions and methods for producing a stonewashed look on indigo-dyed denim fabric and garments. For the manufacture of clothes, the fabric can be cut and sewn into clothes or garments, which are afterwards finished. In particular, for the manufacture of denim jeans, different enzymatic finishing methods have been developed. The finishing of denim garment normally is initiated with an enzymatic desizing step, during which garments are subjected to the action of proteolytic enzymes to provide softness to the fabric and make the cotton more accessible to the subsequent enzymatic finishing steps. The serine protease of the present invention can be used in methods of finishing denim garments (*e.g*., a "bio-stoning process"), enzymatic desizing and providing softness to fabrics, and/or finishing process.

One or more subtilisin variant of the present invention can be used to remove proteins from animals and their subsequent degradation or disposal, such as, e.g., feathers, skin, hair, and hide. In some instances, immersion of the animal carcass in a solution comprising one or more subtilisin variant of the present invention can act to protect the skin from damage in comparison to the traditional immersion in scalding water or the defeathering process. In one embodiment, feathers can be sprayed with one or more subtilisin variant of the present invention under conditions suitable for digesting or initiating degradation of the plumage. In some embodiments, the variant can be used in combination with an oxidizing agent.

In some embodiments, the removal of the oil or fat associated with raw feathers can be assisted by one or more subtilisin variant of the present invention. In some embodiments, the one or more subtilisin variant of the present invention is used in compositions for cleaning the feathers as well as to sanitize and partially dehydrate the fibers. In yet other embodiments, the one or more subtilisin variant of the present invention finds use in recovering protein from plumage. In some other embodiments, the one or more subtilisin variant of the present invention is applied in a wash solution in combination with 95% ethanol or other polar organic solvent with or without a surfactant at about 0.5% (v/v). In other embodiment, the one or more subtilisin variant of the present invention may be used alone or in combination in suitable feather processing and proteolytic methods, such as those disclosed in WO2014/013080, WO2014/013081, and WO2014/013082. In some embodiments, the recovered protein can be subsequently used in animal or fish feed.

In still another embodiment, one or more subtilisin variant of the present invention can be used as a component of an animal feed composition, animal feed additive and/or pet food comprising one or more subtilisin variant present invention. are Also disclosed herein are methods for preparing such an animal feed composition, animal feed additive composition and/or pet food comprising mixing the one or more subtilisin variant of the present invention with one or more animal feed ingredients and/or animal feed additive ingredients and/or pet food ingredients.

The term "animal" includes all non-ruminant and ruminant animals. In a particular embodiment, the animal is a non-ruminant animal, such as a horse and a mono-gastric animal. Examples of mono-gastric animals include, but are not limited to, pigs and swine, such as piglets, growing pigs, sows; poultry such as turkeys, ducks, chicken, broiler chicks, layers; fish such as salmon, trout, tilapia, catfish and carps; and crustaceans such as shrimps and prawns. In a further embodiment, the animal is a ruminant animal including, but not limited to, cattle, young calves, goats, sheep, giraffes, bison, moose, elk, yaks, water buffalo, deer, camels, alpacas, llamas, antelope, pronghom and nilgai.

In the present context, it is intended that the term "pet food" is understood to mean a food for a household animal such as, but not limited to, dogs, cats, gerbils, hamsters, chinchillas, fancy rats, guinea pigs; avian pets, such as canaries, parakeets, and parrots; reptile pets, such as turtles, lizards and snakes; and aquatic pets, such as tropical fish and frogs.

The terms "animal feed composition," "feedstuff" and "fodder" are used interchangeably and can comprise one or more feed materials selected from a) cereals, such as small grains (e.g., wheat, barley, rye, oats and combinations thereof) and/or large grains such as maize or sorghum; b) by products from cereals, such as corn gluten meal, Distillers Dried Grain Solubles (DDGS) (particularly corn based Distillers Dried Grain Solubles (cDDGS), wheat bran, wheat middlings, wheat shorts, rice bran, rice hulls, oat hulls, palm kernel, and citrus pulp; c) protein obtained from sources such as soya, sunflower, peanut, lupin, peas, fava beans, cotton, canola, fish meal, dried plasma protein, meat and bone meal, potato protein, whey, copra, sesame; d) oils and fats obtained from vegetable and animal sources; and e) minerals and vitamins.

one or more subtilisin variant described herein find further use in the enzyme aided bleaching of paper pulps such as chemical pulps, semi-chemical pulps, kraft pulps, mechanical pulps or pulps prepared by the sulfite method. In general terms, paper pulps are incubated with one or more subtilisin variant described herein under conditions suitable for bleaching the paper pulp.

In some instances, the pulps are chlorine free pulps bleached with oxygen, ozone, peroxide or peroxyacids. In some instances, the one or more subtilisin variant described herein is used in enzyme aided bleaching of pulps produced by modified or continuous pulping methods that exhibit low lignin contents. In some other instances, the one or more subtilisin variant described herein is applied alone or preferably in combination with xylanase and/or endoglucanase and/or alpha-galactosidase and/or cellobiohydrolase enzymes.

### EXAMPLE 1

### Assays

### Performance Index

The performance index (PI) of an enzyme compares the performance of the variant (measured value) with the parent enzyme (theoretical value or measured value) at the same protein concentration. Theoretical concentrations for the parent enzyme can be calculated using the parameters extracted from a Langmuir fit of a standard curve of the parent enzyme. A PI that is greater than 1 (PI>1) indicates improved performance by a variant as compared to a parent or benchmark protease (defined for each analyzed data set), while a PI of 1 (PI=1) identifies a variant that performs the same as the parent, and a PI that is less than 1 (PI<1) identifies a variant that performs worse than the parent.

### Protein Determination Assay

Protein concentration determination for BspE04637-T1 parent protease (SEQ ID NO: 1) and BspE04637 variants obtained from culture supernatant was performed with an Agilent U-HPLC system. A calibration curve (0 - 500 ppm) using purified BspE04637-T1 protein was generated and used to generate a standard curve. Aliquots of clarified culture supernatants were diluted 10 fold in dilution buffer (Tris 25mM, pH 7.4, 5mM CaCl₂) and then mixed on a 1:1 ratio with an Acetonitrile containing buffer (22.5mM Tris, pH 7.4, 4.5mM CaCl₂, 9% acetonitrile). These samples were filtered on 45µm filter plates and filtrate was loaded via an auto-sampler onto a reverse phase column (Zorbax 300 SB-C3 column). A linear elution gradient of Buffer A (0.1% Trifluoroacatic acid) and Buffer B (0.07% Acetonitrile was used. At a flow rate of 1 mL/min with a 4 min run time and a 1 min post-run column equilibration. Absorbance was measured at 220 nm, and peaks were integrated using ChemStation software (Agilent Technologies). The protein concentration of the samples was calculated based on the standard curve of the purified BspE04637-T1 parent enzyme.

### Protease Activity Assays

The protease activity of BspE04637-T1 parent protease (SEQ ID NO: 1) and variants thereof was tested by measuring hydrolysis of N-suc-AAPF-pNA, dimethyl casein (DMC).

For the AAPF assay, the reagent solutions used were: 100 mM Tris pH 8.6, 10 mM CalCl₂, 0.005% Tween^{®}-80 (Tris/Ca buffer) and 160 mM suc-AAPF-pNA in DMSO (suc-AAPF-pNA stock solution) (Sigma: S-7388). To prepare a working solution, 1 mL suc-AAPF-pNA stock solution was added to 100 mL Tris/Ca buffer and mixed. An enzyme sample was added to a microtiter plate (MTP) containing 1 mg/mL suc-AAPF-pNA working solution and assayed for activity at 405 nm over 3 minutes using a SpectraMax plate reader in kinetic mode at RT. The protease activity was expressed as mOD/min.

For the DMC assay, the reagent solutions used were: 2.5% Dimethylcasein (DMC, Sigma) in 100 mM Sodium Carbonate pH 9.5, 0.075% TNBSA (2,4,6-trinitrobenzene sulfonic acid, Thermo Scientific) in Reagent A. Reagent A: 45.4 g Na₂B₄O₇.10H₂0 (Merck) in 15 mL 4N NaOH to reach a final volume of 1000 mL in MQ water, Dilution Solution: 10 mM NaCl, 0.1 mM CaCl₂, 0.005% Tween-80, 0.02% Na-azide. MTPs were filled with 47.5 uL DMC substrate following the addition of 2.5 uL of 20 ppm protease supernatant. 50 uL of TNBSA in Reagent A was then added with slow mixing. Activity was measured at 405 nm over 5 minutes using a SpectraMax plate reader in kinetic mode at RT. As stated above for the AAPF assay, activity was expressed as mOD/min.

### Detergents compositions evaluated

Proteases were tested for cleaning performance and/or stability in custom-made liquid detergent formulations ("Test HDL1 and Test HDL2") having the composition set forth in Tables 1.1 and 1.2. The HDL detergents, Test HDL1 and Test HDL 2 are considered boron-free since they contained ≤5mg/Kg of boron, when tested for elemental boron content.

| **Table 1.1: Composition of Test HDL1 Detergent Formula** | | | |
|---|---|---|---|
| **Component** | **Ingredient** | **Trade name** | **Wt.%** |
| Solvent | Water (total) | - | 64.89 |
| Surfactant | C12-C15 Pareth-7 | Empilan KCL 7 | 3 |
| | (Sodium) Dodecylbenzenesulfonate | NANSA SSA F | 7.5 |
| | K-Cocoate | NANSA PC 38F | 3 |
| | Sodium Laureth Sulfate | Empicol ESB3/M6 | 9 |
| Builder | Sodium Citrate | Sodium Citrate Tribasic Dihydrate | 3 |
| Liquid properties /stability | Sorbitol | D-Sorbitol | 0.8 |
| | Propylene glycol | 1,2-Propanediol | 2.5 |
| | Glycerin | Glycerol | 0.8 |
| | Triethanolamine | Triethanolamine | 0.5 |
| | Methylisothiazolinone | 2-Methyl-4-isothiazolin-3 -one | 0.01 |
| | Ethanol | - | 1 |
| Neutralizer | Sodium hydroxide (4M) | - | 5.27 |

| **Table 1.2: Composition of Test HDL2 Detergent Formula** | | | |
|---|---|---|---|
| **Component** | **Ingredient** | **Trade name** | **Wt.%** |
| Solvent | Water (total) | - | QS |
| | Propylene glycol | - | 5 |
| Surfactant | Sodium Alkyl Aryl Ether Sulfate | Polystep B27 (30% active) | 12 |
| | Sodium alkylbenzene sulfonate | Nacconal 90G | 1.2 |
| Builder | Trisodium citrate | - | 0.5 |
| | Disodium ethylenediaminetatraacetate | - | 0.25 |
| Liquid properties /stability | Sodium formate | - | 0.25 |
| | Acrylic acid homopolymer | Sokalan PA 30 CL (50% active) | 0.5 |
| | Oleic acid | - | 1 |
| | Calcium chloride | - | 0.1 |
| | Brightener | Tinopal CBS-X | 0.25 |
| | Preservative | Bioban 425 (25% active) | 0.04 |
| Neutralizer | Sodium carbonate | - | 0.25 |
| | Sodium Hydroxide (50%) to pH 8.5 | - | 0.25 |

### Cleaning Performance in Detergent

Variants were tested for cleaning performance relative to BspE04637-T1 parent protease on various microswatches: BMI (blood/milk/ink on cotton, EMPA-116), egg yolk with pigment (aged by heat, CFT CS-38), and POM (pigment/oil/milk, CFT-C10). Pre-punched (to fit on MTP), rinsed, and filled swatch-containing plates (Corning 3641) were prepared by Center for Testmaterials BV, Vlaardingen, Netherlands. Detergents were dosed as described in Table 1.3, and were added to the MTPs prior to enzyme addition.

| **Table 1.3: List of Wash Conditions Used for Performance Assays** | | | | |
|---|---|---|---|---|
| **Detergent** | **Final Wash Conc, (g/L)** | **Hardness Conc. (ppm)** | **Buffer** | **Set pH** |
| Test HDL1 | 7.5 | 250 | 5 mM sodium HEPES | 8 |
| Test HDL2 | 0.84 | 150 | 5 mM sodium HEPES | 8 |

Aliquots of enzyme were added to the detergent-filled MTPs to reach a final volume of 200 uL with a final enzyme concentration of 1.25 ppm in the wash. Laundry cleaning assays were carried out at 25°C. When testing CS-10 and CS-38 stains using Test HDL1, the assay was carried out for 25 min. When testing EMPA-116 in Test HDL1 or CS-10 and CS-38 in Test HDL2, tests were carried out over 50 min. When EMPA-116 was tested in Test HDL2, the assay was carried out over 65 min. Following these incubations, 130 uL aliquots of each supernatant was transferred to a fresh MTP and a SpectraMax plate reader was used to measure the absorbance at 600 nm for EMPA-116 swatches and at 405 nm for CFT CS-38 and CFT CS-10 swatches. Absorbance results were obtained by subtracting the value for a blank (no enzyme added) from each sample value. The cleaning PI for each assay condition was obtained by dividing the absorbance values for a given variant by that of the BspE0463 7 - T1 parent protease tested at the same concentration.

### Thermostability Assay in Detergent

The variants were tested for stability under stress conditions using 10% Test HDL1 detergent, diluted in deionized water (hereinafter "stressor") by measuring the residual activity following incubation at elevated temperature. The elevated temperature was set to obtain approximately 30% residual activity of the stressed sample compared to the unstressed (baseline) sample.

For the unstressed condition, the diluted enzyme sample was mixed in 10% Test HDL1 detergent (diluted in deionized water) and baseline protease activity (no elevated temperature stress) was measured immediately upon dilution by the DMC assay described above. For the stressed condition, these enzyme samples diluted in stressor were incubated in a 96-well plate at 72°C for 5 min using a thermal cycler. The plate was then cooled and the resulting stressed protease activity was measured by the DMC method. Percent (%) residual activities for each sample were calculated by determining the ratio of the stressed over the baseline protease activity and multiplying the value by 100.

### EXAMPLE 2

### Generation of BspE04637-clade Subtilisins

DNA manipulations to generate BspE04637-clade subtilisins were carried out via molecular biology techniques known in the art. The BspE04637-clade subtilisins are more fully described in WO2016/069557. The predicted and observed sequences of the naturally occurring BspE04637 wild-type protease are set forth in WO2016/069557. A series of artificial DNA sequences were synthesized (by Integrated DNA Technologies, Inc.) to generate the genes encoding the truncated version of the BspE04637 protease sequence of BspE04637-T1 (SEQ ID NO: 1). Molecular biology techniques known in the art were used to introduce multiple amino acid modifications into this sequence, which consists of a C-terminal truncation of the naturally occurring sequence, yielding a mature protein of 278 amino acids (SEQ ID NO:1).

A DNA fragment containing the following elements: the BPN' terminator, chloramphenicol resistance marker from *Staphylococcus aureus* (CAT), *B. subtilis* aprE promoter, *B. subtilis* aprE signal peptide sequence, and native BspE04637 protease pro-peptide was constructed. The pool of genes of interest (encoding the various BspE04637-T1 variants) and the DNA fragment described above were assembled using Gibson technology to form an integration cassette that targets the *B. Subtilis aprE* locus. These expression cassettes consisted of: *B. subtilis* aprE promoter (SEQ ID NO:2), the *B. subtilis* aprE signal peptide sequence (SEQ ID NO:3 for nucleotide sequence and SEQ ID NO:4 for the amino acid sequence), the native BspE04637 protease pro-peptide (SEQ ID NO: 5 for nucleotide sequence and SEQ ID NO: 6 for amino acid sequence), the region encoding the mature protease sequences for BspE04637-T1 parent (SEQ ID NO:1) or variants thereof, and a *B amyloliquefaciens* terminator (SEQ ID NO:7). The nucleotide sequences of the BspE04637-T1 variants are set forth in SEQ ID NOs: 8-17 and the amino acid sequences of the BspE04637-T1 variants are set forth in SEQ ID NOs: 18-27.

A suitable *B. subtilis* strain was transformed with the reaction products of the above mentioned Gibson assembly reaction and the transformed cells were plated on Luria's Agar + 5 ppm chloramphenicol containing skim milk. Single colonies from the agar plates were picked into individual wells of a 96 well MTP that contained 150 microliters of Luria's broth + 5 ppm chloramphenicol in each well. The cultures were grown overnight at 37°C and 250 RPM. 75 microliters of 50% glycerol (diluted in water) was added to each well and the MTPs for long-term storage at -80°C.

To produce the recombinant proteases, the *B. subtilis* carrying each gene of interest was cultivated in an enriched semi-defined media for 2 days at 37°C and 250 RPM. Cultures were subjected to centrifugation and filtration, and the clarified supernatants were used for the screening assays described below.

### EXAMPLE 3

### Evaluation of BspE04637-clade Protease Variants for Cleaning and Stability

BspE04637-clade protease variants listed in Table 4 were tested for cleaning performance relative to BspE04637-T1 parent protease on the following microswatches: BMI (EMPA-116), egg yolk with pigment (aged by heat, CFT CS-38), and POM (pigment/oil/milk, CFT-C10).

| **Table 4: BspE04637-clade Protease Variants** | | | |
|---|---|---|---|
| **Sample ID** | **Sequence Substitutions Relative to BspE04637-T1 Parent** | **No. Mutations vs Parent** | **Amino Acid Sequence** |
| BSPE-01299 | R37A-D53G-S101R-N120R-I143R-D166N-D184S-P197K-L238K-E248Q-D255N-R272N | 12 | SEQ ID NO:18 |
| BSPE-01314 | D15K-D53R-N56K-S105K-D122H-D166N-D184S-P197K-E248Q-D255N-V274E | 11 | SEQ ID NO:19 |
| BSPE-01329 | D15K-A55R-S101R-N120R-I143R-D166N-D184S-P197K-L238K-E248Q-N268K-V274E | 12 | SEQ ID NO:20 |
| BSPE-01346 | H48R-A55R-S101R-N120R-S 133R-R164N-L238K-E248Q-D255N-N268K | 10 | SEQ ID NO:21 |
| BSPE-01420 | Q12K-D15K-R37A-D122H-R164N-G169D-E248Q-N268K-D275S | 9 | SEQ ID NO:22 |
| BSPE-01443 | D15K-H48R-A55R-S101R-N120R-I143R-G165R-D175N-A198E-N243R-N254K-D275K | 12 | SEQ ID NO:23 |
| BSPE-01483 | Q12K-V26R-R37A-A55R-N78D-S105K-I143R-A155V-D184S-A198E-N243R-N259R-D275K | 13 | SEQ ID NO:24 |
| BSPE-01560 | T14D-D53R-S101R-I143R-R164N-D175S-A198E-N243R-D275K | 9 | SEQ ID NO:25 |
| BSPE-01579 | D15K-D53R-S101R-N120R-I143R-G165R-D175S-N243R-D255N-N264K-D275K | 11 | SEQ ID NO:26 |
| BSPE-01581 | Q12K-V26R-R37A-H48R-A55R-N78D-S101R-D122H-I143R-D166N-D175P-D184S-Y220R-N243R-D255N-R272N | 16 | SEQ ID NO:27 |

Table 5 shows the results obtained when the cleaning of various BspE04637-clade protease variants were compared to BspE04637-T1 parent protease in Test HDL1 and Test HDL2 detergents.

| **Table 5: Cleaning of BspE04637-clade Protease Variants Compared to BspE04637-T1 Parent Protease, Reported as PI** | | | | | | |
|---|---|---|---|---|---|---|
| | **EMPA-116** | | **CFT CS-38** | | **CFT C-10** | |
| **Sample ID** | **Test HDL1** | **Test HDL2** | **Test HDL1** | **Test HDL2** | **Test HDL1** | **Test HDL2** |
| BSPE-01443 | 1.83 | 3.56 | 1.18 | 3.24 | 1.87 | 2.05 |
| BSPE-01581 | 2.06 | 3.44 | 1.18 | 2.47 | 1.84 | 2.08 |
| BSPE-01483 | 2.15 | 3.37 | 1.26 | 3.10 | 1.85 | 1.84 |
| BSPE-01560 | 2.24 | 3.68 | 1.19 | 2.97 | 1.79 | 1.84 |
| BSPE-01314 | 2.26 | 3.89 | 1.29 | 3.47 | 1.92 | 2.23 |
| BSPE-01346 | 1.75 | 3.54 | 1.24 | 3.35 | 1.74 | 1.99 |
| BSPE-01329 | 1.84 | 3.76 | 1.24 | 3.11 | 1.75 | 2.04 |
| BSPE-01299 | 2.02 | 3.67 | 1.26 | 2.62 | 1.91 | 2.01 |
| BspE04637-T1 parent | 1.00 | 1.00 | 1.00 | 1.00 | 1.00 | 1.00 |

Table 6 shows the stability of various BspE04637 protease variants in 10% Test HDL 1 compared to BspE04637-T1 parent protease as measured in the Stability Assay described in Example 1 hereinabove.

| **Table 6: Stability of BspE04637-T1 and Variants, Reported as % Residual Activity** | |
|---|---|
| **Sample ID** | **% Residual Activity in 10% Test HDL 1 (72°C for 5 min)** |
| BSPE-01579 | 60 |
| BSPE-01420 | 52 |
| BSPE-01443 | 31 |
| BSPE-01581 | 31 |
| BSPE-01483 | 19 |
| BSPE-01560 | 20 |
| BSPE-01314 | 22 |
| BSPE-01346 | 14 |
| BSPE-01329 | 19 |
| BSPE-01299 | 16 |
| BspE04637-T1 | 51 |

### EXAMPLE 4

### Sequence Alignment of BspE04637-T1 and Variants Thereof

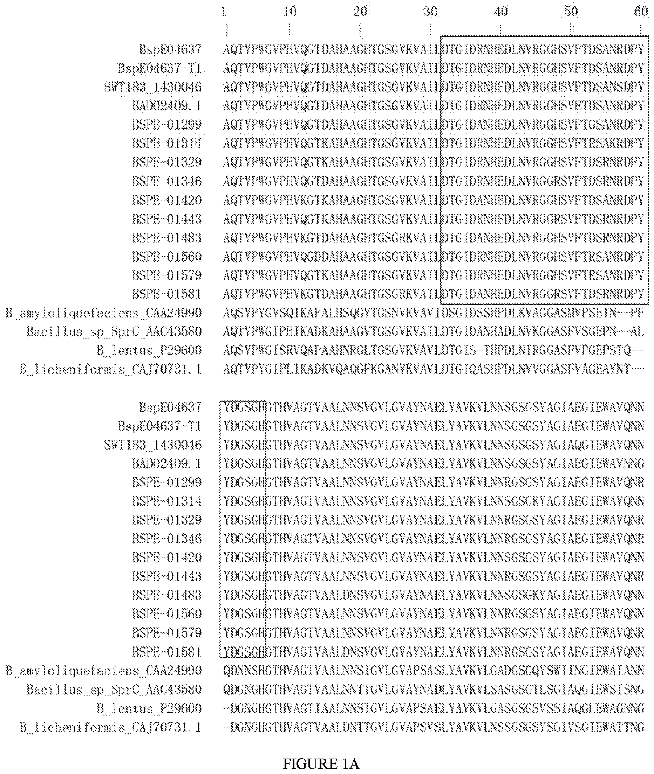

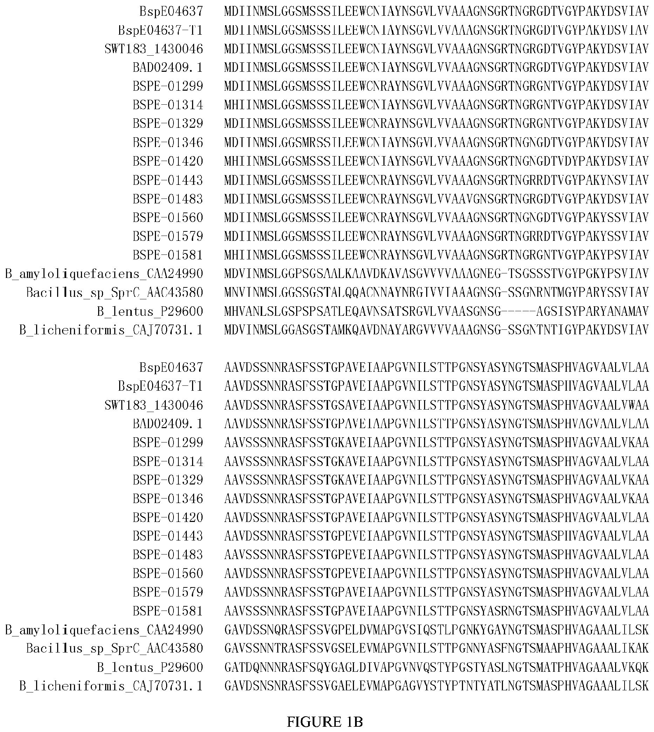

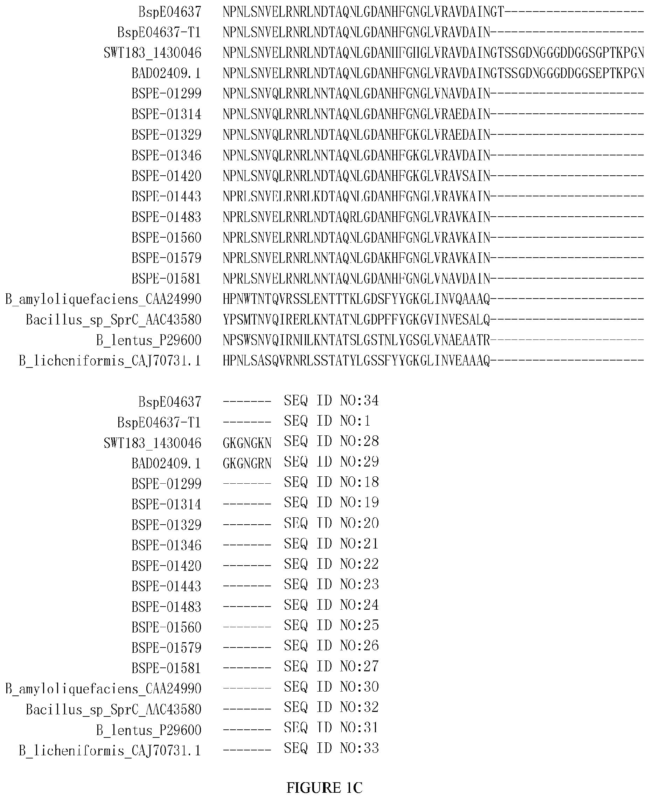

An alignment of the amino acid sequences of BspE04637-T1 parent protease (SEQ ID NO:1) and protease variants thereof: BSPE-01299 (SEQ ID NO:18), BSPE-01314 (SEQ ID NO:19), BSPE-01329 (SEQ ID NO: 20), BSPE-01346 (SEQ ID NO:21), BSPE-01420 (SEQ ID NO:22), BSPE-01443 (SEQ ID NO:23), BSPE-01483 (SEQ ID NO:24), BSPE-01560 (SEQ ID NO:25), BSPE-01579 (SEQ ID NO:26), and BSPE-01581 (SEQ ID NO:27) with the naturally truncated form of BspE04637 (SEQ ID NO:34) (which is further described in WO2016/069557) previously disclosed BspE04637-clade subtilisins: SWT183_1430046 (SEQ ID NO: 28) and *Bacillus sp.* KSM-LD1_ BAD02409.1 (SEQ ID NO: 29); and the sequences of the mature forms of the following subtilisins: *Bacillus amyloliquefaciens*_CAA24990 (SEQ ID NO: 30), *Bacillus lentus*_P29000 (SEQ ID NO: 31), *Bacillus sp.* SprC _AAC43580 (SEQ ID NO: 32), and *Bacillus licheniformis_*CAJ70731 (SEQ ID NO: 33), is shown in Figure 1. A box has been drawn around the motif of the BspE04637-clade proteases. The sequences were aligned with default parameters using the MUSCLE program from Geneious software (Biomatters Ltd.) (Robert C. Edgar, MUSCLE: multiple sequence alignment with high accuracy and high throughput, Nucl. Acids Res. (2004) 32 (5): 1792-1797).

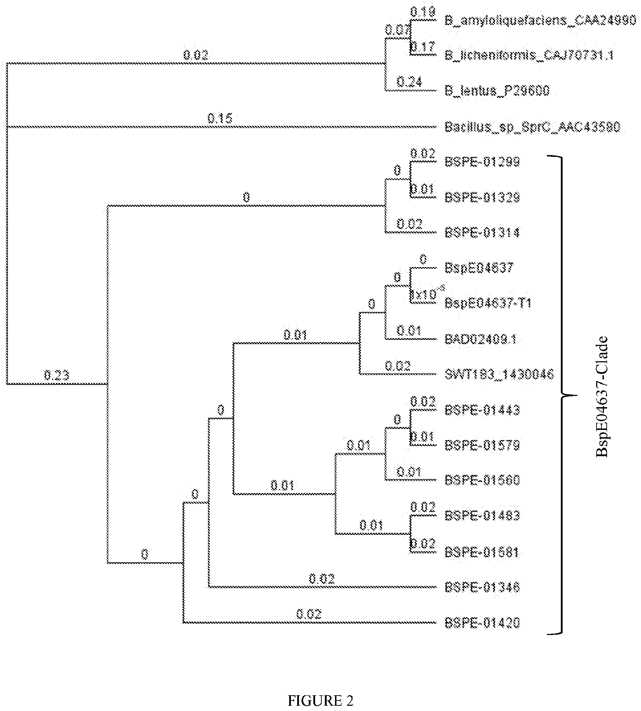

A phylogenetic tree for amino acid sequences of the mature forms of the subtilisins from Figure 1 was built using the Geneious Tree builder program, and is set forth in Figure 2.

## Claims

1. An subtilisin variant comprising an amino acid sequence having at least 90% sequence identity to the amino acid sequence of SEQ ID NO: 1 or 34 and comprising an amino acid substitution X120R wherein the amino acid positions of the variant are numbered by correspondence with the amino acid sequence of SEQ ID NO: 1, and wherein said variant has protease activity, wherein said variant has improved cleaning performance when compared to a reference subtilisin comprising an amino acid sequence of SEQ ID NO: 1 or 34, wherein said variant has a BMI (blood/milk/ink on cotton), POM (pigment/oil/milk) and/or egg yolk with pigment swatch cleaning PI >1, optionally wherein cleaning performance in detergent is measured in accordance the cleaning performance in detergent assay of Example 1.

2. The subtilisin variant of claim 1, wherein said variant comprises an amino acid sequence additionally comprising one, two, three, or four or more amino acid substitutions selected from X12K, X14D, X15K, X26R, X37A, X48R, X53G/R, X55R, X56K, X78D, X101R, X105K, X122H, X133R, X143R, X155V, X164N, X165R, X166N, X169D, X175N/P/S, X184S, X197K, X198E, X220R, X238K, X243R, X248Q, X254K, X255N, X259R, X264K, X268K, X272N, X274E, and X275S/K wherein X is any amino acid.

3. The subtilisin variant of any preceding claim, with the proviso that the
(i) amino acid substitution at position 197 is not an S;
(ii) amino acid substitution at position 238 is not a W;
(iii) amino acid substitution at position 268 is not H;
(iv) said variant does not consist of the amino acid sequence of BAD02409 or JP2003325186-0001; and/or
(v) said variant does not consist of the amino acid sequence of SWT183_1430046.

4. The subtilisin variant of claim 1 or 2, wherein said variant comprises an amino acid sequence comprising one, two, three, or four or more amino acid substitutions selected from:
(i) Q12K, T14D, D15K, V26R, R37A, H48R, D53G/R, A55R, N56K, N78D, S101R, S105K, G/N120R, D122H, S133R, I143R, A155V, R164N, G165R, D166N, G169D, D175N/P/S, D184S, P/S197K, A198E, T220R, L/W238K, N243R, E248Q, N254K, D255N, N259R, N264K, H/N268K, R272N, V274E, and D275S/K; or
(ii) Q12K, T14D, D15K, V26R, R37A, H48R, D53G/R, A55R, N56K, N78D, S101R, S105K, N120R, D122H, S133R, I143R, A155V, R164N, G165R, D166N, G169D, D175N/P/S, D184S, P197K, A198E, T220R, L238K, N243R, E248Q, N254K, D255N, N259R, N264K, N268K, R272N, V274E, and D275S/K.

5. The subtilisin variant of any preceding claim, wherein said variant comprises an amino acid sequence comprising nine or more amino acid substitutions selected from: R37A-D53G-S101R-N120R-I143R-D166N-D184S-P197K-L238K-E248Q-D255N-R272N; D15K-A55R-S101R-N120R-I143R-D166N-D184S-P197K-L238K-E248Q-N268K-V274E; H48R-A55R-S101R-N120R-S133R-R164N-L238K-E248Q-D255N-N268K; D15K-H48R-A55R-S101R-N120R-I143R-G165R-D175N-A198E-N243R-N254K-D275K; D15K-D53R-S101R-N120R-I143R-G165R-D175S-N243R-D255N-N264K-D275K; and combinations thereof.

6. The subtilisin variant of any preceding claim, wherein said variant further comprises one or more motif selected from a:
i. DTGIXXXHXDLXXXGGXSVFXXXXXXXXXXDXXGH (SEQ ID NO 35 ) motif, wherein the initial D is the active site Aspartic acid, the terminal H is the active site Histidine, and X is any amino acid;
ii.DTGIXXXHXDLXXXGGXSVFXXXXXXDPXXDXXGH (SEQ ID NC 36) motif, wherein the initial D is the active site Aspartic acid, the terminal H is the active site Histidine, and X is any amino acid;
iii. DTGIXXXHXDLNVXGGXSVFXXXXXXXXXXDXXGH (SEQ ID NC 37) motif, wherein the initial D is the active site Aspartic acid, the terminal H is the active site Histidine, and X is any amino acid;
iv. DTGIXXXHXDLNVXGGXSVFXXXXXXDPXXDXXGH (SEQ ID NO 38) motif, wherein the initial D is the active site Aspartic acid, the terminal H is the active site Histidine, and X is any amino acid;
v.DTGIDXXHXDLNVXGGXS VFXXXXXXXXXXDXXGH (SEQ ID NO 39) motif, wherein the initial D is the active site Aspartic acid and the terminal H is the active site Histidine, and X is any amino acid;
vi. DTGIDXNHXDL NVRGGXSVFTXXXXX DPXXDXXGH (SEQ ID NO 40) motif, wherein the initial D is the active site Aspartic acid, the terminal H is the active site Histidine, and X is any amino acid; and
vii. DTGIDXNHXDLNVRGGXSVFTXXXXX DPYYDXXGH (SEQ ID NO 41) motif, wherein the initial D is the active site Aspartic acid and the terminal H is the active site Histidine, and X is any amino acid.

7. The subtilisin variant of any preceding claim, wherein said variant:
(a) comprises an amino acid sequence having at least 95% amino acid sequence identity to an amino acid sequence of SEQ ID NO:1 or 34, and/or
(b) is isolated; and/or
(c) has one or more additional improved properties when compared to a reference subtilisin, wherein the improved property is selected from improved protease activity, and improved thermostability in detergent, wherein the reference subtilisin comprises an amino acid sequence of SEQ ID NO:1 or 34.

8. The subtilisin variant of claim 7, wherein the additional improved property is
(i) improved protease activity, wherein said variant has a PI > 1 on N-suc-AAPF-pNA or dimethyl casein substrate, optionally wherein protease activity is measured in accordance with the protease activity assay of Example 1;
and/or
(ii) improved thermostability, wherein said variant has a stability PI > 1, optionally wherein thermostability in detergent is measured in accordance with the thermostability in detergent assay of Example 1.

9. A composition comprising one or more subtilisin variant of any preceding claim.

10. The composition of claim 9, wherein said composition is a detergent composition, optionally wherein the detergent composition is selected from the group consisting of a laundry detergent, a fabric softening detergent, a dishwashing detergent, and a hard-surface cleaning detergent.

11. The composition of any one of claims 9-10, wherein said composition
(a) further comprises at least one ion selected from calcium and zinc; at least one stabilizer; from about 0.001% to about 1.0 weight % of said variant; at least one bleaching agent; at least one adjunct ingredient; and/or one or more additional enzymes or enzyme derivatives selected from the group consisting of acyl transferases, alpha-amylases, beta-amylases, alpha-galactosidases, arabinosidases, aryl esterases, beta-galactosidases, carrageenases, catalases, cellobiohydrolases, cellulases, chondroitinases, cutinases, endo-beta-1,4-glucanases, endo-beta-mannanases, esterases, exo-beta-mannanases, galactanases, glucoamylases, hemicellulases, hyaluronidases, keratinases, laccases, lactases, ligninases, lipases, lipoxygenases, mannanases, oxidases, pectate lyases, pectin acetyl esterases, pectinases, pentosanases, perhydrolases, peroxidases, phenoloxidases, phosphatases, phospholipases, phytases, polygalacturonases, additional proteases, pullulanases, reductases, rhamnogalacturonases, beta-glucanases, tannases, transglutaminases, xylan acetyl-esterases, xylanases, xyloglucanases, xylosidases, metalloproteases, and combinations thereof; and/or.
(b) contains phosphate or is phosphate-free and/or contains borate or is borate-free; and/or
(c) is a granular, powder, solid, bar, liquid, tablet, gel, paste or unit dose composition.

12. The composition of claim 9, wherein said composition is an animal feed, contact lens cleaning, wound cleaning, or textile, leather or feather processing composition.

13. A method of cleaning, comprising contacting a surface or an item in need of cleaning with the variant of any one of claims 1-8 or the composition of any one of claims 9-11; and optionally further comprising the step of rinsing said surface or item after contacting said surface or item with said variant or composition, optionally wherein said item is dishware or fabric.

14. A polynucleotide comprising a nucleic acid sequence that encodes the variant of any one of claims 1-8, wherein said polynucleotide is optionally isolated.

15. An expression vector or cassette comprising the polynucleotide of claim 14, optionally wherein the nucleic acid sequence is operably linked to a promoter.

16. A recombinant host cell comprising the vector or cassette of claim 15 or the polynucleotide of claim 14.

## Patentansprüche

1. Subtilisin-Variante, die eine Aminosäuresequenz umfasst, welche eine mindestens 90 %ige Sequenzübereinstimmung mit der Aminosäuresequenz der SEQ ID Nr. 1 oder 34 aufweist und eine Aminosäuresubstitution X120R umfasst, wobei die Aminosäurepositionen der Variante derart nummeriert sind, dass sie der Aminosäuresequenz der SEQ ID Nr.: 1 entsprechen, und wobei die Variante eine Proteaseaktivität hat, wobei die Variante eine verbesserte Reinigungsleistung aufweist, wenn sie mit einem Referenz-Subtilisin verglichen wird, welches eine Aminosäuresequenz der SEQ ID Nr.: 1 oder 34 umfasst, wobei die Variante für BMI (Blut/Milch/Druckfarbe), POM (Pigment/Öl/Milch) und/oder Eigelb beim Reinigen eines pigmentierten Stoffmusters einen PI-Wert von >1 hat, wobei die Reinigungsleistung in Reinigungsmitteln möglicherweise gemäß der Versuchsreihe zur Reinigungsleistung in Reinigungsmitteln des Beispiels 1 gemessen wird.

2. Subtilisin-Variante nach Anspruch 1, wobei die Variante eine Aminosäurevariante umfasst, die zusätzlich eine, zwei, drei oder vier oder mehr Aminosäuresubstitutionen umfasst, welche aus X12K, X14D, X15K, X26R, X37A, X48R, X53G/R, X55R, X56K, X78D, X101R, X105K, X122H, X133R, X143R, X155V, X164N, X165R, X166N, X169D, X175N/P/S, X184S, X197K, X198E, X220R, X238K, X243R, X248Q, X254K, X255N, X259R, X264K, X268K, X272N, X274E und X275S/K ausgewählt sind, wobei X eine beliebige Aminosäure ist.

3. Subtilisin-Variante nach einem beliebigen vorhergehenden Anspruch, mit der Maßgabe, dass
(i) es sich bei der Aminosäuresubstitution an Position 197 nicht um ein S handelt;
(ii) es sich bei der Aminosäuresubstitution an Position 238 nicht um ein W handelt;
(iii) es sich bei der Aminosäuresubstitution an Position 268 nicht um H handelt;
(iv) die Variante nicht in der Aminosäuresequenz von BAD02409 oder JP2003325186-0001 besteht; und/oder
(v) die Variante nicht in der Aminosäuresequenz von SWT183_1430046 besteht.

4. Subtilisin-Variante nach Anspruch 1 oder 2, wobei die Variante eine Aminosäuresequenz umfasst, welche eine, zwei, drei oder vier oder mehr Aminosäuresubstitutionen umfasst, die aus den folgenden ausgewählt sind:
(i) Q12K, T14D, D15K, V26R, R37A, H48R, D53G/R, A55R, N56K, N78D, S101R, S105K, G/N120R, D122H, S133R, I143R, A155V, R164N, G165R, D166N, G169D, D175N/P/S, D184S, P/S197K, A198E, T220R, L/W238K, N243R, E248Q, N254K, D255N, N259R, N264K, H/N268K, R272N, V274E und D275S/K; oder
(ii) Q12K, T14D, D15K, V26R, R37A, H48R, D53G/R, A55R, N56K, N78D, S101R, S105K, N120R, D122H, S133R, I143R, A155V, R164N, G165R, D166N, G169D, D175N/P/S, D184S, P197K, A198E, T220R, L238K, N243R, E248Q, N254K, D255N, N259R, N264K, N268K, R272N, V274E und D275S/K.

5. Subtilisin-Variante nach einem beliebigen vorhergehenden Anspruch, wobei die Variante eine Aminosäuresequenz umfasst, welche neun oder mehr Aminosäuresubstitutionen umfasst, die aus den folgenden ausgewählt sind: R37A-D53G-S101R-N120R-I143R-D166N-D184S-P197K-L238K-E248Q-D255N-R272N; D15K-A55R-S101R-N120R-I143R-D166N-D184S-P197K-L238K-E248Q-N268K-V274E; H48R-A55R-S101R-N120R-S133R-R164N-L238K-E248Q-D255N-N268K; D15K-H48R-A55R-S101R-N120R-I143R-G165R-D175N-A198E-N243R-N254K-D275K; D15K-D53R-S101R-N120R-I143R-G165R-D175S-N243R-D255N-N264K-D275K; und Kombinationen derselben.

6. Subtilisin-Variante nach einem beliebigen vorhergehenden Anspruch, wobei die Variante weiterhin einen oder mehrere Bausteine umfasst, die aus den folgenden ausgewählt sind:
i. einem Baustein DTGIXXXHXDLXXXGGXSVFXXXXXXXXXXDXXGH (SEQ ID Nr. 35), wobei es sich bei dem D am Anfang um die Asparaginsäure der aktiven Stelle handelt, und es sich bei dem H am Ende um das Histidin der aktiven Stelle handelt, und X für eine beliebige Aminosäure steht;
ii. einem Baustein DTGIXXXHXDLXXXGGXSVFXXXXXXDPXXDXXGH (SEQ ID Nr. 36), wobei es sich bei dem D am Anfang um die Asparaginsäure der aktiven Stelle handelt, und es sich bei dem H am Ende um das Histidin der aktiven Stelle handelt, und X für eine beliebige Aminosäure steht;
iii. einem Baustein DTGIXXXHXDLNVXGGXSVFXXXXXXXXXXDXXGH (SEQ ID Nr. 37), wobei es sich bei dem D am Anfang um die Asparaginsäure der aktiven Stelle handelt, und es sich bei dem H am Ende um das Histidin der aktiven Stelle handelt, und X für eine beliebige Aminosäure steht;
iv. einem Baustein DTGIXXXHXDLNVXGGXSVFXXXXXXDPXXDXXGH (SEQ ID Nr. 38), wobei es sich bei dem D am Anfang um die Asparaginsäure der aktiven Stelle handelt, und es sich bei dem H am Ende um das Histidin der aktiven Stelle handelt, und X für eine beliebige Aminosäure steht;
v. einem Baustein DTGIDXXHXDLNVXGGXS VFXXXXXXXXXXDXXGH (SEQ ID Nr. 39), wobei es sich bei dem D am Anfang um die Asparaginsäure der aktiven Stelle handelt, und es sich bei dem H am Ende um das Histidin der aktiven Stelle handelt, und X für eine beliebige Aminosäure steht;
vi. einem Baustein DTGIDXNHXDL NVRGGXSVFTXXXXX DPXXDXXGH (SEQ ID Nr. 40), wobei es sich bei dem D am Anfang um die Asparaginsäure der aktiven Stelle handelt, und es sich bei dem H am Ende um das Histidin der aktiven Stelle handelt, und X für eine beliebige Aminosäure steht; und
vii. einem Baustein DTGIDXNHXDLNVRGGXSVFTXXXXX DPYYDXXGH (SEQ ID Nr. 41), wobei es sich bei dem D am Anfang um die Asparaginsäure der aktiven Stelle handelt, und es sich bei dem H am Ende um das Histidin der aktiven Stelle handelt, und X für eine beliebige Aminosäure steht.

7. Subtilisin-Variante nach einem beliebigen vorhergehenden Anspruch, wobei die Variante:
(a) eine Aminosäuresequenz umfasst, welche eine mindestens 95 %ige Sequenzübereinstimmung mit einer Aminosäuresequenz der SEQ ID Nr. 1 oder 34 aufweist, und/oder
(b) isoliert wird; und/oder
(c) eine oder mehrere zusätzliche verbesserte Eigenschaften im Vergleich zu einem Referenz-Subtilisin aufweist, wobei die verbesserte Eigenschaft aus einer verbesserten Protease-Aktivität und einer verbesserten Hitzestabilität in Reinigungsmitteln ausgewählt ist, wobei das Referenz-Subtilisin eine Aminosäuresequenz der SEQ ID Nr. 1 oder 34 umfasst.

8. Subtilisin-Variante nach Anspruch 7, wobei es sich bei der zusätzlichen verbesserten Eigenschaft um Folgendes handelt
(i) eine verbesserte Protease-Aktivität, wobei die Variante einen PI > 1 bei Einwirkung auf ein N-suc-AAPF-pNA- oder ein Dimethylkasein-Substrat hat, wobei die Protease-Aktivität möglicherweise gemäß der Versuchsreihe zur Protease-Aktivität des Beispiels 1 gemessen wird;
und/oder
(ii) eine verbesserte Hitzestabilität, wobei die Variante eine Stabilität PI > 1 aufweist, wobei die Hitzestabilität in Reinigungsmitteln möglicherweise gemäß der Versuchsreihe zur Hitzestabilität in Reinigungsmitteln des Beispiels 1 gemessen wird.

9. Zusammensetzung, die eine oder mehrere Subtilisin-Varianten nach einem beliebigen vorhergehenden Anspruch umfasst.

10. Zusammensetzung nach Anspruch 9, wobei es sich bei der Zusammensetzung um eine Reinigungsmittelzusammensetzung handelt, wobei die Reinigungsmittelzusammensetzung möglicherweise aus der Gruppe ausgewählt ist, welche aus einem Wäschewaschmittel, einem Weichspüler, einem Geschirrspülmittel und einem Reinigungsmittel für harte Oberflächen besteht.

11. Zusammensetzung nach einem beliebigen der Ansprüche 9 bis 10, wobei die Zusammensetzung
(i) weiterhin mindestens ein Ion, das aus Calcium und Zink ausgewählt ist; mindestens einen Stabilisator; ungefähr 0,001 % bis ungefähr 1,0 Gewichts-% der Variante; mindestens ein Bleichmittel; mindestens einen Zusatzstoff; und/oder ein oder mehrere zusätzliche Enzyme oder Enzymderivate umfasst, welche der Gruppe ausgewählt sind, die aus den Acyltransferasen, alpha-Amylasen, beta-Amylasen, alpha-Galactosidasen, Arabinosidasen, Arylesterasen, beta-Galactosidasen, Carrageenasen, Catalasen, Cellobiohydrolasen, Cellulasen, Chondroitinasen, Cutinasen, endo-beta-1,4-Glucanasen, endo-beta-Mannanasen, Esterasen, exo-beta-Mannanasen, Galactanasen, Glucoamylasen, Hemicellulasen, Hyaluronidasen, Keratinasen, Laccasen, Lactasen, Ligninasen, Lipasen, Lipoxygenasen, Mannanasen, Oxidasen, Pectatlyasen, Pectinacetyl-Esterasen, Pectinasen, Pentosanasen, Perhydrolasen, Peroxidasen, Phenoloxidasen, Phosphatasen, Phospholipasen, Phytasen, Polygalacturonasen, zusätzlichen Proteasen, Pullulanasen, Reductasen, Rhamnogalacturonasen, beta-Glucanasen, Tannasen, Transglutaminasen, Xylanacetyl-Esterasen, Xylanasen, Xyloglucanasen, Xylosidasen, Metalloproteasen und deren Kombinationen besteht; und/oder
(b) Phosphat enthält oder phosphatfrei ist und/oder Borat enthält oder boratfrei ist; und/oder
(c) als Granulat, Pulver, Feststoff, Riegel, Flüssigkeit, Tablette, Gel, Paste oder Einheitsdosiszusammensetzung vorliegt.

12. Zusammensetzung nach Anspruch 9, wobei es sich bei der Zusammensetzung um ein Tierfuttermittel, ein Kontaktlinsenreinigungsmittel, ein Wundreinigungsmittel oder eine Zusammensetzung zur Verarbeitung von Textilien, Leder oder Federn handelt.

13. Reinigungsverfahren, im Rahmen dessen eine Oberfläche oder ein Gegenstand, der/die einer Reinigung bedarf, mit der Variante nach einem beliebigen der Ansprüche 1 bis 8 oder der Zusammensetzung nach einem beliebigen der Ansprüche 9 bis 11 in Kontakt gebracht wird; wobei es möglicherweise weiterhin den Schritt des Spülens der Oberfläche oder des Gegenstands umfasst, nachdem die Oberfläche oder der Gegenstand mit der Variante oder der Zusammensetzung in Kontakt gebracht wurde, wobei es sich bei dem Gegenstand möglicherweise um Geschirr und Textilstoff handelt.

14. Polynukleotid, das eine Nukleinsäuresequenz umfasst, welche für die Variante nach einem beliebigen der Ansprüche 1 bis 8 codiert, wobei das Polynukleotid möglicherweise isoliert wird.

15. Expressionsvektor oder -kassette, welcher/welche das Polynukleotid nach Anspruch 14 umfasst, wobei die Nukleinsäuresequenz funktionsfähig mit einem Promotor verknüpft ist.

16. Rekombinante Wirtszelle, welche den Vektor oder die Kassette nach Anspruch 15 oder das Polynukleotid nach Anspruch 14 umfasst.

## Revendications

1. Variant de subtilisine comprenant une séquence d'acides aminés ayant une identité de séquence d'au moins 90 % avec la séquence d'acides aminés de SEQ ID NO: 1 ou 34 et comprenant une substitution d'acides aminés X120R, dans lequel les positions d'acides aminés du variant sont numérotées par correspondance avec la séquence d'acides aminés de SEQ ID NO: 1, et dans lequel ledit variant a une activité de protéase, ledit variant ayant une performance améliorée de nettoyage par comparaison avec une subtilisine de référence comprenant une séquence d'acides aminés de SEQ ID NO: 1 ou 34, ledit variant ayant un PI > 1 pour le nettoyage d'un petit échantillon BMI (sang/lait/encre sur coton), POM (pigment/huile/lait) et/ou jaune d'œuf sur pigment, éventuellement la performance de nettoyage dans le détergent étant mesurée conformément à la performance de nettoyage dans l'essai de détergent de l'Exemple 1.

2. Variant de subtilisine selon la revendication 1, ledit variant comprenant une séquence d'acides aminés comprenant en outre un, deux, trois ou quatre ou plus substitutions d'acides aminés choisies parmi X12K, X14D, X15K, X26R, X37A, X48R, X53G/R, X55R, X56K, X78D, X101R, X105K, X122H, X133R, X143R, X155V, X164N, X165R, X166N, X169D, X175N/P/S, X184S, X197K, X198E, X220R, X238K, X243R, X248Q, X254K, X255N, X259R, X264K, X268K, X272N, X274E et X275S/K, K étant un acide aminé quelconque.

3. Variant de subtilisine selon l'une quelconque des revendications précédentes, à la condition que
(i) la substitution d'acides aminés en position 197 ne soit pas un S ;
(ii) la substitution d'acides aminés en position 238 ne soit pas un W ;
(iii) la substitution d'acides aminés en position 268 ne soit pas H ;
(iv) ledit variant ne consiste pas en la séquence d'acides aminés de BAD02409 ou JP2003325186-0001 ; et/ou
(v) ledit variant ne consiste pas en la séquence d'acides aminés de SWT183_1430046.

4. Variant de subtilisine selon la revendication 1 ou 2, ledit variant comprenant une séquence d'acides aminés comprenant une, deux, trois ou quatre ou plus substitutions d'acides aminés choisies parmi
(i) Q12K, T14D, D15K, V26R, R37A, H48R, D53G/R, A55R, N56K, N78D, S101R, S105K, G/N120R, D122H, S133R, I143R, A155V, R164N, G165R, D166N, G169D, D175N/P/S, D184S, P/S197K, A198E, T220R, L/W238K, N243R, E248Q, N254K, D255N, N259R, N264K, H/N268K, R272N, V274E et D275S/K ; ou
(ii) Q12K, T14D, D15K, V26R, R37A, H48R, D53G/R, A55R, N56K, N78D, S101R, S105K, N120R, D122H, S133R, I143R, A155V, R164N, G165R, D166N, G169D, D175N/P/S, D184S, P197K, A198E, T220R, L238K, N243R, E248Q, N254K, D255N, N259R, N264K, N268K, R272N, V274E et D275S/K.

5. Variant de subtilisine selon l'une quelconque des revendications précédentes, ledit variant comprenant une séquence d'acides aminés comprenant neuf ou plus substitutions d'acides aminés choisies parmi R37A-D53G-S101R-N120R-I143R-D166N-D184S-P197K-L238K-E248Q-D255N-R272N ; D15K-A55R-S101R-N120R-I143R-D166N-D184S-P197K-L238K-E248Q-N268K-V274E ; H48R-A55R-S101R-N120R-S133R-R164N-L238K-E248Q-D255N-N268K ; D15K-H48R-A55R-S101R-N120R-I143R-G165R-D175N-A198E-N243R-N254K-D275K ; D15K-D53R-S101R-N120R-I143R-G165R-D175S-N243R-D255N-N264K-D275K ; et les combinaisons de celles-ci.

6. Variant de subtilisine selon l'une quelconque des revendications précédentes, ledit variant comprenant en outre un ou plusieurs motifs choisis parmi
i. un motif DTGIXXXHXDLXXXGGXSVFXXXXXXXXXXDXXGH (SEQ ID NO: 35), dans lequel le D initial représente le site actif acide aspartique, le H terminal représente le site actif histidine et X représente un acide aminé quelconque ;
ii. un motif DTGIXXXHXDLXXXGGXSVFXXXXXXDPXXDXXGH (SEQ ID NO: 36), dans lequel le D initial représente le site actif acide aspartique, le H terminal représente le site actif histidine et X représente un acide aminé quelconque ;
iii. un motif DTGIXXXHXDLNVXGGXSVFXXXXXXXXXXDXXGH (SEQ ID NO: 37), dans lequel le D initial représente le site actif acide aspartique, le H terminal représente le site actif histidine et X représente un acide aminé quelconque ;
iv. un motif DTGIXXXHXDLNVXGGXSVFXXXXXXDPXXDXXGH (SEQ ID NO: 38), dans lequel le D initial représente le site actif acide aspartique, le H terminal représente le site actif histidine, et X représente un acide aminé quelconque ;
v. un motif DTGIDXXHXDLNVXGGXS VFXXXXXXXXXXDXXGH (SEQ ID NO: 39), dans lequel le D initial représente le site actif acide aspartique et le H terminal représente le site actif histidine, et X représente un acide aminé quelconque ;
vi. un motif DTGIDXNHXDL NVRGGXSVFTXXXXX DPXXDXXGH (SEQ ID NO: 40), dans lequel le D initial représente le site actif acide aspartique, le H terminal représente le site actif histidine, et X représente un acide aminé quelconque ; et
vii. un motif DTGIDXNHXDLNVRGGXSVFTXXXXX DPYYDXXGH (SEQ ID NO: 41), dans lequel le D initial représente le site actif acide aspartique et le H terminal représente le site actif histidine, et X représente un acide aminé quelconque.

7. Variant de subtilisine selon l'une quelconque des revendications précédentes, ledit variant
(a) comprenant une séquence d'acides aminés ayant une identité de séquence d'acides aminés d'au moins 95 % avec une séquence d'acides aminés de SEQ ID NO: 1 ou 34, et/ou
(b) est isolé ; et/ou
(c) a une ou plusieurs propriétés améliorées additionnelles par comparaison avec une subtilisine de référence, la propriété améliorée étant choisie parmi une activité de protéase améliorée et une thermostabilité améliorée dans le détergent, la subtilisine de référence comprenant une séquence d'acides aminés de SEQ ID NO: 1 ou 34.

8. Variant de subtilisine selon la revendication 7, dans lequel la propriété améliorée additionnelle est
(i) une activité de protéase améliorée, ledit variant ayant un PI > 1 sur un substrat de N-suc-AAPF-pNA ou de diméthyl caséine, éventuellement l'activité de protéase étant mesurée conformément à l'essai d'activité de protéase de l'Exemple 1 ;
et/ou
(ii) une thermostabilité améliorée, ledit variant ayant une stabilité PI > 1, éventuellement la thermostabilité dans le détergent étant mesurée conformément à la thermostabilité dans l'essai de détergent de l'Exemple 1.

9. Composition comprenant un ou plusieurs variants de subtilisine selon l'une quelconque des revendications précédentes.

10. Composition selon la revendication 9, ladite composition étant une composition détergente, éventuellement la composition détergente étant choisie dans le groupe consistant en un détergent pour le linge, un détergent assouplissant textile, un détergent pour lave-vaisselle et un détergent pour le nettoyage des surfaces dures.

11. Composition selon l'une quelconque des revendications 9 à 10, ladite composition
(a) comprenant en outre au moins un ion choisi parmi le calcium et le zinc ; au moins un stabilisant ; d'environ 0,001 % à environ 1,0 % en poids dudit variant ; au moins un agent de blanchiment ; au moins un ingrédient succédané ; et/ou une ou plusieurs enzymes ou un ou plusieurs dérivés d'enzymes additionnels choisis dans le groupe consistant en les acyl transférases, les alpha-amylases, les bêta-amylases, les alpha-galactosidases, les arabinosidases, les aryl estérases, les bêta-galactosidases, les carragénases, les catalases, les cellobiohydrolases, les cellulases, les chondroïtinases, les cutinases, les endo-bêta-1,4-glucanases, les endo-bêta-mannanases, les estérases, les exo-bêta-mannanases, les galactanases, les glucoamylases, les hémicellulases, les hyaluronidases, les kératinases, les laccases, les lactases, les ligninases, les lipases, les lipoxygénases, les mannanases, les oxydases, les pectate lyases, les pectine acétyl estérases, les pectinases, les pentosanases, les perhydrolases, les peroxydases, les phénoloxydases, les phosphatases, les phospholipases, les phytases, les polygalacturonases, les protéases additionnelles, les pullulanases, les réductases, les rhamnogalacturonases, les bêta-glucanases, les tannases, les transglutaminases, les xylane acétyl-estérases, les xylanases, les xyloglucanases, les xylosidases, les métalloprotéases et les combinaisons de celles-ci ; et/ou
(b) contenant un phosphate ou étant exempt de phosphate et/ou contenant un borate ou étant exempt de borate ; et/ou
(c) étant une composition granulaire, en poudre, solide, en barre, liquide, en comprimé, en gel, en pâte ou en dose unitaire.

12. Composition selon la revendication 9, ladite composition étant une composition d'aliment pour animaux, de nettoyage de lentille de contact, de parage des plaies, ou de traitement de textiles, de cuir ou de plumes.

13. Procédé de nettoyage, comprenant la mise en contact d'une surface ou d'une pièce ayant besoin d'être nettoyée avec le variant selon l'une quelconque des revendications 1 à 8 ou avec la composition selon l'une quelconque des revendications 9 à 11 ; et éventuellement comprenant en outre l'étape de rinçage de ladite surface ou de ladite pièce après mise en contact de ladite surface ou de ladite pièce avec ledit variant ou ladite composition, éventuellement ladite pièce étant un article de vaisselle ou un tissu.

14. Polynucléotide comprenant une séquence d'acides nucléiques qui code pour le variant selon l'une quelconque des revendications 1 à 8, ledit polynucléotide étant éventuellement isolé.

15. Vecteur ou cassette d'expression comprenant le polynucléotide selon la revendication 14, éventuellement la séquence d'acides nucléiques étant fonctionnellement liée à un promoteur.

16. Cellule hôte recombinante comprenant le vecteur ou la cassette selon la revendication 15 ou le polynucléotide selon la revendication 14.
